(19) Europäisches Patentamt
European Patent Office
Office européen des brevets

(11) **EP 4 516 304 A1**

(12) **EUROPEAN PATENT APPLICATION**
published in accordance with Art. 153(4) EPC

(43) Date of publication:
**05.03.2025 Bulletin 2025/10**

(21) Application number: **23807473.6**

(22) Date of filing: **08.05.2023**

(51) International Patent Classification (IPC):
*A61K 35/74* (2015.01)    *A61K 50/00* (2006.01)
*A61P 35/00* (2006.01)    *A61P 37/04* (2006.01)
*C12N 1/20* (2006.01)

(52) Cooperative Patent Classification (CPC):
A61K 9/0009; A61K 35/74; A61P 35/00;
A61P 37/04; C12N 1/20

(86) International application number:
**PCT/JP2023/017320**

(87) International publication number:
**WO 2023/223869 (23.11.2023 Gazette 2023/47)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **19.05.2022 JP 2022082458**

(71) Applicant: **Japan Advanced Institute of Science
and Technology
Ishikawa 923-1292 (JP)**

(72) Inventor: **MIYAKO Eijiro
Nomi-shi, Ishikawa 923-1292 (JP)**

(74) Representative: **Forrest, Stuart
WP Thompson
138 Fetter Lane
London EC4A 1BT (GB)**

Remarks:
The complete document including Reference
Table(s) and the Sequence Listing(s) can be
downloaded from the EPO website

(54) **ANTITUMOR AGENT, IMAGE GENERATION METHOD, BACTERIUM, AND PRODUCTION METHOD**

(57) [Summary]

[Problems] The purpose of the present invention is to provide a technology useful for the treatment or diagnosis of a cancer.

[Solution] The present invention provides an antitumor agent comprising a purple non-sulfur bacterium isolated from a tumor. The present invention also provides a method for generating an image for identifying a tumor, comprising: an administration step of administering a bacterium isolated from a tumor to an animal; and an image generation step of, after the administration, capturing an image of the animal under irradiation with light in a predetermined wavelength range to generate an image for identifying a tumor that may be present in the animal. The present invention also provides a bacterium isolated from a tumor, comprising at least a purple non-sulfur bacterium.

[Fig. 1]

## Description

Technical Field

The present invention relates to an antitumor agent, an image generation method, a bacterium, and a production method. In particular, the present invention relates to an antitumor agent comprising a bacterium isolated from a tumor, an image generation method using a bacterium isolated from a tumor, a bacterium isolated from a tumor, and a bacterium production method comprising a step of isolating a bacterium from a tumor.

Background Art

In order to treat cancer, various treatment methods are used, such as surgery, drug therapy, and radiation therapy. These may be used alone or in combination depending on the type and condition of the disease. Drugs such as anticancer drugs are used in drug therapy. Anticancer drugs used in drug therapy may also have adverse effects on normal cells, which may be a heavy burden for patients. For example, anticancer drugs accumulate in patients through long-term administration, and often cause significant side effects to patients.

Also, early detection of cancer through testing is one of the important factors for improving the prognosis of patients. For example, various tumor markers, MRI, and CT are used for cancer testing. The presence or location of cancer cannot be determined by the value of the tumor marker alone. Although MRI and CT are very useful for identifying the presence or absence of cancer, these devices are relatively large and costly.

The following Patent Literature 1 discloses the use of photosynthetic bacteria for the treatment or diagnosis of cancer. For example, the document describes administering photosynthetic bacteria to a subject, allowing the photosynthetic bacteria to accumulate in an affected area where cancer is present, and then irradiating the affected area with light.

Citation List

Patent Literature

Patent Literature 1: International Publication WO2022/025043

Summary of Invention

Technical Problem

An object of the present invention is to provide a technique that is useful for treating or diagnosing cancer.

Solution to Problem

Various bacteria are present in tumors. The present inventors isolated bacteria present in tumors from the tumors and found that the isolated bacteria are useful for cancer treatment or diagnosis, or both.

That is, the present invention provides the following:

[1] An antitumor agent comprising a purple non-sulfur bacterium isolated from a tumor.

[2] The antitumor agent according to [1], wherein the purple non-sulfur bacterium is a bacterium of the genus Rhodopseudomonas or a bacterium of the genus Blastochloris, or both of these.

[3] The antitumor agent according to [1] or [2], which is administered parenterally.

[4] The antitumor agent according to any one of [1] to [3], further comprising, in addition to the purple non-sulfur bacteria, other bacteria isolated from tumors.

[5] The antitumor agent according to [4], wherein the other bacteria are bacteria of the genus Proteus.

[6] The antitumor agent according to [4] or [5], wherein the antitumor agent contains the purple non-sulfur bacteria and the other bacteria in a ratio of 1CFU:99CFU to 99CFU:1CFU.

[7] The antitumor agent according to any one of [1] to [6], wherein the bacterium contained in the antitumor agent has an immune cell stimulation property.

[8] The antitumor agent according to any one of [1] to [7], wherein the bacterium contained in the antitumor agent has an expression enhancing property that enhances the expression of an antitumor biomarker in a tumor.

[9] The antitumor agent according to any one of [1] to [8], wherein the bacterium contained in the antitumor agent exhibits the following property regarding the absorption spectrum:

<Property regarding absorption spectrum>
when measuring the absorption spectrum of a dispersion liquid in which the bacterium contained in the antitumor agent is dispersed in a PBS buffer at a concentration of $2\times10^7$ CFU/mL, the absorbance at 808 nm is 0.029 or more.

[10] The antitumor agent according to any one of [1] to [9], wherein the bacterium contained in the antitumor agent exhibits the following property regarding the fluorescence spectrum:

<Property regarding fluorescence spectrum>
when measuring the fluorescence spectrum of a dispersion liquid in which the bacterium contained in the antitumor agent is dispersed in a PBS buffer at a concentration of $2.5\times10^7$ CFU/mL, the fluorescence intensity at 888 nm is 4.4 or more.

[11] A method for generating an image for tumor identification, comprising:

an administration step of administering one or more types of bacteria isolated from a tumor to an animal; and an image generation step of capturing an image of the animal after the administration while irradiated with light in a predetermined wavelength range to generate an image for identifying any tumors that may be present in the animal.

[12] A bacterium isolated from a tumor, comprising at least a purple non-sulfur bacterium.
[13] The bacterium according to [12], wherein the bacterium isolated from a tumor further comprises a bacterium of the genus Proteus.
[14] The bacterium according to [12] or [13], wherein the purple non-sulfur bacterium is a bacterium of the genus Rhodopseudomonas or a bacterium of the genus Blastochloris, or both.
[15] The bacterium according to any one of [12] to [14], which exhibits the following property regarding the absorption spectrum:

<Property regarding absorption spectrum>
when measuring the absorption spectrum of a dispersion liquid in which the bacterium is dispersed in a PBS buffer at a concentration of $2\times10^7$ CFU/mL, the absorbance at 808 nm is 0.029 or more.

[16] The bacterium according to any one of [12] to [15], which exhibits the following property regarding the fluorescence spectrum:

<Property regarding fluorescence spectrum>
when measuring the fluorescence spectrum of a dispersion liquid in which the bacterium is dispersed in a PBS buffer at a concentration of $2.5\times10^7$ CFU/mL, the fluorescence intensity at 888 nm is 4.4 or more.

[17] A bacterium production method, comprising:

an isolation step of isolating bacteria from a tumor; and
a culturing step of culturing the bacteria isolated in the isolation step in a medium not containing cysteine added;
wherein the cultured bacteria comprise at least a purple non-sulfur bacterium and have antitumor activity.

Advantageous Effects of Invention

Bacteria isolated from tumors are very useful for tumor treatment. For example, tumors can be treated by the antitumor agent according to the present invention. The antitumor agent according to the present invention can also be used to treat cancers in various organs or tissues.
Bacteria isolated from tumors are also very useful for tumor diagnosis. For example, the method according to the invention (particularly, the image generation method) or the bacteria according to the invention are useful for identifying the presence or absence of a tumor, and further for identifying the location or shape of a tumor.
Furthermore, the present invention also allows for simultaneous treatment and diagnosis of a tumor. For example, administration of an antitumor agent according to the present invention can not only treat a tumor but also identify the location of the tumor.
The effects of the present invention are not limited to those described herein, and may be any of the effects described in this specification.

Brief Description of Drawings

[Fig. 1] A view for illustrating the isolation and culture of intratumoral bacteria.

[Fig. 2A] A view for illustrating in vivo anticancer experiments.

[Fig. 2B] Photographs of mice after intravenous administration of each bacteria.

[Fig. 2C] A graph showing the results of evaluating the antitumor activity of seed bacteria.

[Fig. 2D] A graph showing mouse survival rates for 40 days after cancer cell transplantation for each bacterial administration.

[Fig. 2E] A graph showing the rate of complete response in each experimental group.

[Fig. 2F] A view showing the experimental method for investigating the acquisition of cancer immunity.

[Fig. 2G] Photographs showing the observation results of mice after re-transplantation of Colon-26 are shown.

[Fig. 2H] A graph showing the results of measuring the tumor volume in each treatment group.

[Fig. 2I] A graph showing the results of measuring the absorption spectrum.

[Fig. 2J] A graph showing the measurement results of the fluorescence spectrum.

[Fig. 2K] A view showing images obtained by in vivo fluorescence bioimaging.

[Fig. 3A] A view showing the staining results of immune cells.

[Fig. 3B] A view showing the staining results of antitumor biomarkers.

[Fig. 3C] A view showing the results of colony assay when i-R.P. was administered.

[Fig. 3D] A view showing the results of colony assay when i-P.M. was administered.

[Fig. 3E] A view showing the results of H&E histological staining of the various organs when i-P.M., i-R.P., or PBS was administered.

[Fig. 4A] A view explaining a method for producing a sarcoma model mouse.

[Fig. 4B] A graph showing changes in tumors over time in sarcoma model mice.

[Fig. 4C] A view explaining a method for producing a metastatic lung cancer model mouse.

[Fig. 4D] Photographs of lungs excised from each mouse.

[Fig. 4E] A graph showing the results of measuring the weight of lungs excised from mice.

[Fig. 4F] A graph showing changes in mouse body weight over time.

[Fig. 5] A view showing the results of measuring the absorption spectrum and the fluorescence spectrum.

[Fig. 6] A view showing photographs of mice and fluorescent observation images obtained by in vivo fluorescent bioimaging.

Description of Embodiments

The present invention will be described in the following order.

1. Overview of the Invention

1.1 Antitumor activity
1.2 Diagnostic usefulness
1.3 Usefulness from the viewpoint of toxicity
1.4 Usefulness from the viewpoint of conferring cancer immunity
1.5 Addressing the problems with proposed treatment methods

2. Embodiments

2.1 First embodiment (antitumor agent)

2.1.1 Antitumor agent containing purple non-sulfur bacteria isolated from tumors
2.1.2 Antitumor agent containing bacteria of the genus Proteus isolated from tumors
2.1.3 Other bacterial examples
2.1.4 Composition of antitumor agent
2.1.5 Method for producing antitumor agent

2.2 Second embodiment (bacteria)

2.2.1 Purple non-sulfur bacteria

2.2.2 Bacteria of the genus Proteus

2.3 Third embodiment (image generation method)
2.4 Fourth embodiment (bacterium production method)
2.5 Fifth embodiment (treatment method and diagnosis method)

3. Example

It should be noted that the present invention is not limited to these embodiments, and can be freely modified within the scope of the present invention.

1. Overview of the Invention

As described above, various treatment methods are used to treat cancer, such as surgical therapy, drug therapy, and radiation therapy. In addition, various proposals have been made regarding the cancer treatment methods.

Nanomedicine, for example, has been proposed as a method for cancer treatment. One example of such nanomedicine is one that utilizes the EPR effect. Unlike normal vascular endothelial cells, there are wide gaps of about 200 nm between vascular endothelial cells in cancer tissues and inflammatory sites, and the EPR effect (Enhanced Permeation and Retention Effect) is known, in which nanoparticles with a size controlled to about 10 to 100 nm accumulate specifically in cancer tissues. In many cases, anti-cancer treatments using nanotechnology are close to the performance limit because they rely on this EPR effect. In fact, it has been reported that there are several environmental factors that inhibit solid cancers, and the EPR effect itself is not useful (for example, Masayuki Yokoyama, Drug Delivery System, 33(2), p. 89-97 (2018)). In addition, drug carriers using the EPR effect have not been successful in human clinical trials.

Thus, nanomedicine relies on the EPR effect, which has an unclear mechanism, and has low selectivity against cancer. Another problem with nanomedicine is that it uses anticancer drugs that have strong side effects. Nanomedicine also requires a great deal of time, effort, and cost to synthesize. In addition, because the inside of a tumor is in an oxygen-deficient state, photodynamic therapy-type nanomedicine that uses oxygen is less effective.

Antibody therapy has also been proposed as a method for cancer treatment. For example, a strategy has been proposed in which cancer cell-specific antibodies or the like are loaded onto drug carriers to improve tumor targeting performance (see, for example, Hisataka Kobayashi, Drug Delivery System, 29(4), p. 274-284 (2014)). However, large antibodies have difficulty in penetrating the stromal barrier surrounding cancer, and sufficient selectivity and efficacy have not been achieved.

Thus, because antibodies cannot penetrate the cancer stromal barrier (they only bind to the surface of the tumor), it is difficult to eliminate cancer cells deep inside the tumor, even though immune cells are used. In addition, for antibody therapy, it is necessary to create order (owner) made antibodies for biomarkers that are expressed at different levels between cancer patients. In addition, it requires a great deal of time, effort, and cost to create the drug itself that is equipped with an antibody.

In recent years, bacteriotherapy has also been proposed as a method for cancer treatment, and in particular, cancer targeted therapy using anaerobic microorganisms that can selectively accumulate, grow, and proliferate inside tumors under hypoxic conditions has attracted attention (Shibin Zhou et al. Nature Reviews Cancer, 18, p. 727-743 (2018)). However, conventional cancer bacteriotherapy is basically a concept of a drug delivery system, which is the transportation of anticancer drugs. In addition, in order to express anticancer activity, it is necessary to manipulate or modify microorganisms using genetic engineering. As a result, unpredictable and uncontrollable problems such as bacteria acquiring drug resistance may occur, and it is said that application to medical treatment is difficult. In addition, the bacteria used are often Salmonella or Escherichia coli that have been attenuated by genetic modification, and there is always a risk that they will become virulent again in the body.

Thus, for bacteriotherapy, the use of Salmonella, Listeria, or Escherichia coli that have been attenuated by genetic modification has been proposed, but these have the risk of becoming virulent again (reverting mutation) in the body. In addition, genetic modification may lead to the acquisition of drug resistance. Furthermore, tumor specificity is insufficient, and it may also accumulate in normal organs or tissues. In addition, the above bacteriotherapy is a passive drug delivery method, and requires complicated genetic design.

1.1 Antitumor activity

The present inventors have found that by isolating intratumor-present bacteria from the tumor, the bacteria exhibit excellent antitumor activity.

Various bacteria are present in tumors. It is believed that these bacteria do not attack the tumor when present in the tumor. However, the present inventors have found that such bacteria present in the tumor can exert antitumor activity when isolated from the tumor. That is, the present invention provides an antitumor agent containing bacteria isolated from a tumor.

In one embodiment, the bacteria isolated from the tumor are purple non-sulfur bacteria. The purple non-sulfur bacteria isolated from the tumor have excellent antitumor activity. In particular, the purple non-sulfur bacteria isolated from the tumor and cultured in a medium exhibit excellent antitumor activity. The purple non-sulfur bacteria may be, for example, a bacterium of the genus Rhodopseudomonas or a bacterium of the genus Blastchloris, or both of them. The antitumor activity is very excellent and is effective against tumors in various organs or tissues.

In the above embodiment, the purple non-sulfur bacteria may be preferably used in combination with other bacteria isolated from tumors. The combination of the purple non-sulfur bacteria isolated from tumors and other bacteria isolated from tumors exhibits particularly excellent antitumor activity. The other bacteria may be, for example, bacteria of the genus Proteus. The two or more types of bacteria constituting the combination are preferably isolated from the same tumor and may be cultured together (co-cultured) in the same medium.

In another embodiment, the bacteria isolated from the tumor are bacteria of the genus Proteus. The bacteria of the genus Proteus isolated from the tumor may be used in combination with the purple non-sulfur bacteria as described above, but may also be used alone. The bacteria of the genus Proteus isolated from the tumor have excellent antitumor activity. The antitumor activity is very excellent and is effective against tumors in various organs or tissues.

1.2 Diagnostic usefulness

The present inventors have also found that the bacteria isolated from the tumor described above have the property of gathering at the tumor site. For example, the bacteria isolated from the tumor gather at the tumor site when parenterally administered to an animal. Therefore, for example, performing an analysis using the optical property of the bacteria is useful for determining the presence or absence of a tumor or for identifying the position or shape of a tumor. For example, information (particularly image information) obtained by capturing an image of the animal using the optical property of the bacteria is useful for the above-mentioned determination or identification. In this way, the bacteria isolated from the tumor are useful not only for tumor treatment but also for tumor diagnosis, and can be used, for example, as a diagnostic medicine. Furthermore, the bacteria isolated from the tumor can be used for both tumor treatment and tumor diagnosis.

In one embodiment, the bacteria isolated from the tumor may be purple non-sulfur bacteria. Purple non-sulfur bacteria isolated from tumors have an optical property different from those of ordinary purple non-sulfur bacteria (e.g., commercially available purple non-sulfur bacteria, etc.). By utilizing the optical property as well as the above-mentioned property of concentrating at the tumor site, it is possible to determine the presence or absence of a tumor or to identify the position or shape of a tumor.

1.3 Usefulness from the viewpoint of toxicity

The present inventors have also found that the above-mentioned bacteria isolated from tumors are superior in terms of toxicity.

In general, when a bacterium is administered to an animal (particularly parenterally), the bacterium often exerts toxicity to the animal. For example, when commercially available purple non-sulfur bacteria or bacteria of the genus Proteus are administered to an animal, these bacteria may exert toxicity to the animal.

However, the bacteria isolated from the tumors described above are not thought to be toxic to animals when administered (particularly parenterally) or, even if they are toxic, the toxicity is thought to be low.

The present inventors have also found that the above-mentioned bacteria isolated from tumors remain in the animal's body for a while after administration, but cannot be detected in the animal's body after a certain period of time has passed. That is, it is supposed that the bacteria isolated from tumors are unlikely to remain in the animal's body for a long period of time, thereby reducing concerns about adverse effects of the bacteria.

1.4 Usefulness from the viewpoint of conferring cancer immunity

The present inventors have also found that the bacteria isolated from the tumors mentioned above confer or enhance immunity against tumors.

For example, the bacteria isolated from the tumors have antitumor activity as described above, so that the bacteria can be administered to an animal with a tumor to cause the tumor to disappear. It was also found that even if the animal is treated to cause a tumor after the tumor disappears, the tumor does not appear in the animal. Therefore, for example, the antitumor agent (particularly, the bacteria isolated from the tumors) may be used to prevent the recurrence of tumors.

1.5 Addressing the problems with proposed treatment methods

The antitumor agent according to the present invention can also address the problems associated with the above-mentioned treatment methods.

For example, nanomedicine, as described above, relies on the EPR effect of an unclear mechanism and has low selectivity against cancer. On the other hand, the antitumor agent (especially bacteria) according to the present invention can exhibit high selectivity against tumors. This high selectivity is thought to be due to, for example, hypoxia, immune evasion, and chemotaxis, i.e., exerted by a clear mechanism.

Furthermore, nanomedicine utilizes anticancer drugs that have strong side effects, but the antitumor agent according to the present invention does not utilize anticancer drugs that have strong side effects, and the bacteria used in the present invention are low toxic.

Furthermore, while nanomedicines require a great deal of time, effort, and cost to synthesize, the bacteria contained in the antitumor agent of the present invention can self-replicate indefinitely, only require cheap food, and are easy to manage.

In order to perform antibody therapy, as described above, it is necessary to create order (owner) made antibodies against biomarkers that have different expression levels among cancer patients. On the other hand, the bacteria contained in the antitumor agent according to the present invention can highly selectively accumulate in the microenvironment common to solid cancers, and can grow and proliferate in the microenvironment. Therefore, there is no need to create antibodies as described above.

In addition, the active ingredient of antibody therapy cannot penetrate the cancer stromal barrier and mainly binds only to the surface of the tumor, so it is difficult to eliminate cancer cells deep inside the tumor, even though immune cells are used. On the other hand, the bacteria contained in the antitumor agent according to the present invention can accumulate deep inside the tumor and grow and proliferate there. Therefore, it is possible to completely eliminate cancer cells from deep inside the tumor.

In addition, the active ingredients of antibody therapy require a great deal of time, effort and cost to produce. On the other hand, the bacteria contained in the antitumor agent of the present invention can self-replicate indefinitely, only require cheap food and are easy to manage.

In conventional bacterial therapy, Salmonella, Listeria, or Escherichia coli that have been attenuated by genetic modification are used, but these bacteria have the risk of becoming virulent again (reverting mutation) in the body, and there is also a possibility of acquiring drug resistance through genetic modification. Furthermore, these bacteria have insufficient tumor specificity and may accumulate in, for example, normal organs or tissues. On the other hand, the bacteria contained in the antitumor agent according to the present invention do not need to be genetically modified, and can also express high selectivity for tumors. Furthermore, the bacteria used are of low toxicity.

In addition, conventional bacteriotherapy is based on passive drug delivery and requires complicated genetic engineering. On the other hand, the bacteria contained in the antitumor agent according to the present invention does not require genetic modification and can eliminate tumors with just a single dose of the bacteria.

In the following, embodiments of the invention are described in more detail.

2. Embodiment

2.1 First embodiment (antitumor agent)

The present invention provides an antitumor agent comprising a bacterium isolated from a tumor. The antitumor agent may comprise, for example, one or more types of bacterium isolated from a tumor, and, for example, the antitumor agent may comprise one type or two types of bacterium isolated from a tumor. In the antitumor agent, the bacterium isolated from a tumor may be used as an active ingredient that exerts antitumor activity.

2.1.1 Antitumor agent containing purple non-sulfur bacteria isolated from tumors

In one embodiment of the present invention, the bacteria isolated from the tumor include a purple non-sulfur bacterium isolated from the tumor. As described above, the purple non-sulfur bacterium isolated from the tumor can exert excellent antitumor activity.

In addition, the purple non-sulfur bacteria accumulate in tumors when administered to an animal (particularly when administered parenterally). Therefore, the antitumor agent does not have to be administered directly to the tumor site, but may be administered directly to the tumor site.

In addition, the purple non-sulfur bacteria have a specific optical property. Therefore, the antitumor agent may be administered for the treatment of a tumor based on the antitumor activity, but may also be used to determine the presence or absence of a tumor or to identify the shape or location of a tumor, in addition to treating the tumor.

(Types of bacteria)

Examples of the purple non-sulfur bacteria include:

bacteria of the genus Rhodopseudomonas, such as Rhodopseudomonas Palustris and Rhodopseudomonas pseudopalustris, etc.;
bacteria of the genus Blastochloris, such as Blastochloris viridis and Blastochloris sulfoviridis, etc.;
bacteria of the genus Afifella, such as Afifella marina, etc.;
bacteria of the genus Rhodobacter, such as Rhodobacter blasticus, Rhodobacter capsulatus, and Rhodobacter sphaeroides, etc.;
bacteria of the genus Rubrivivax, such as Rubrivivax gelatinosus, etc.;
bacteria of the genus Pararhodospirillum, such as Pararhodospirillum oryzae and Pararhodospirillum sulfurexigens, etc.;
bacteria of the genus Rhodocista, such as Rhodocista centenaria, etc.;
bacteria of the genus Marichromatium, such as Marichromatium litoris, etc.;
bacteria of the genus Phaeochromatium, such as Phaeochromatium fluminis, etc.;
bacteria of the genus Rhodoferax, such as Rhodoferax fermentans, etc.;
bacteria of the genus Rhodomicrobium, such as Rhodomicrobium udaipurense and Rhodomicrobium vannielii, etc.; and
bacteria of the genus Rhodovulum, such as Rhodovulum sulfidophilum, etc.

The purple non-sulfur bacteria isolated from tumors contained in the antitumor agent according to the present invention may be, for example, any one of the bacteria of the genera listed above or a combination of two or more thereof.

Furthermore, the purple non-sulfur bacteria isolated from tumors contained in the antitumor agent according to the present invention may be, for example, any one of the bacteria of the species listed above or a combination of two or more thereof.

In a preferred embodiment, the purple non-sulfur bacteria contained in the antitumor agent according to the present invention may be bacteria of the genus Rhodopseudomonas or bacteria of the genus Blastochloris, or both. These bacteria isolated from tumors can exhibit particularly excellent antitumor activity. In addition, these bacteria isolated from tumors have the property of concentrating in tumors when administered to animals (particularly parenterally) and have a specific optical property, so they are also useful for generating tumor diagnostic images.

For example, the purple non-sulfur bacteria isolated from tumors contained in the antitumor agent may be any one, two, three, or four of Rhodopseudomonas Palustris, Rhodopseudomonas pseudopalustris, Blastochloris viridis, and Blastochloris sulfoviridis. Particularly preferably, the purple non-sulfur bacterium contained in the antitumor agent according to the present invention may include at least Rhodopseudomonas Palustris isolated from a tumor. For example, the purple non-sulfur bacterium contained in the antitumor agent according to the present invention may include only Rhodopseudomonas Palustris isolated from a tumor.

In a particularly preferred embodiment, the purple non-sulfur bacterium contained in the antitumor agent according to the present invention is a bacterium of the genus Rhodopseudomonas. The bacteria of the genus Rhodopseudomonas isolated from tumors can exhibit particularly excellent antitumor activity.

For example, the purple non-sulfur bacterium contained in the antitumor agent according to the present invention may be Rhodopseudomonas Palustris or Rhodopseudomonas pseudopalustris, or both of them. Particularly preferably, the purple non-sulfur bacteria contained in the antitumor agent according to the present invention may include at least Rhodopseudomonas Palustris isolated from a tumor.

In a particularly preferred embodiment, the antitumor agent according to the present invention further comprises other bacteria in addition to the purple non-sulfur bacteria. A combination of the purple non-sulfur bacteria isolated from a tumor and the other bacteria isolated from a tumor exhibits particularly excellent antitumor activity. The other bacteria may be, for example, bacteria of the genus Proteus. The two or more types of bacteria constituting the combination may preferably be isolated together from the same tumor and cultured together (co-cultured). That is, the antitumor agent according to the present invention may comprise a combination of the purple non-sulfur bacteria (e.g., the bacteria of the genus Rhodopseudomonas) and the other bacteria (e.g., the bacteria of the genus Proteus) isolated together from a tumor.

The bacterium of the genus Proteus as the other bacterium may be, for example, any one, two or three of Proteus mirabilis, Proteus vulgaris and Proteus myxofaciens, and may be preferably Proteus mirabilis. The combination of the purple non-sulfur bacterium isolated from a tumor and Proteus mirabilis isolated from a tumor exhibits a particularly excellent antitumor effect.

In a particularly preferred embodiment, the antitumor agent may comprise a combination of Rhodopseudomonas Palustris and Proteus mirabilis isolated from a tumor. These two types of complex bacteria exhibit particularly excellent antitumor activity. The complex bacteria may be, for example, a complex bacteria deposited under the accession number: NITE BP-03627. The complex bacteria were deposited internationally at National Institute of Technology and Evaluation, Patent Microorganisms Depositary (NPMD, Room 122, 2-5-8 Kazusa Kamatari, Kisarazu City, Chiba Prefecture, 292-0818) on March 23, 2022. In addition, in the present invention, one of the complex bacteria may be isolated from the other and used.

The composition ratio of the purple non-sulfur bacteria to the other bacteria in the antitumor agent may be, for example, 99:1 to 1:99, but preferably the content ratio of the purple non-sulfur bacteria is higher. In a preferred embodiment, the composition ratio of the purple non-sulfur bacteria to the other bacteria in the antitumor agent may be, for example, 99:1 to 50:50, more preferably 99:1 to 55:45. The composition ratio may be, for example, 80:20 to 55:45, or 70:30 to 60:40. These composition ratios may be based on the CFU of each bacterium.

Such a composition ratio, particularly a higher content ratio of the purple non-sulfur bacteria than the content ratio of the other bacteria, results in excellent antitumor activity.

The constituent ratio based on the CFU can be determined by inoculating the bacteria in the antitumor agent onto a medium in a petri dish and counting the number of colonies formed by culturing for a predetermined period of time.

For example, Proteus mirabilis, which is listed as one of the other bacteria, forms colonies 2 to 3 days after the start of culture, while Rhodopseudomonas Palustris, which is one of the purple non-sulfur bacteria, forms colonies 7 to 10 days after the start of culture. Therefore, the colony counts of Proteus mirabilis and Rhodopseudomonas Palustris can be obtained by counting the number of colonies formed 2 to 3 days after the start of culture, and then counting the number of colonies formed 7 to 10 days after the start of culture, respectively. The constituent ratio may be determined based on these colony counts.

In this way, when the antitumor agent contains the purple non-sulfur bacteria and the other bacteria, the difference in the period required for each bacterium to form a colony is used to determine the composition ratio of these bacteria.

Alternatively, when the periods required for forming colonies are approximately the same, the number of colonies may be counted based on the optical property of the purple non-sulfur bacteria and the other bacteria. The purple non-sulfur bacteria have a specific optical property, but the other bacteria do not have the same optical property. Therefore, it may be determined which bacteria are in the colonies formed on the medium in the petri dish based on the presence or absence of the optical property, and the number of colonies of each bacterium may be counted.

(Optical property of bacteria)

Preferably, the bacteria isolated from the tumor may have an optical property as described below. Bacteria isolated from tumors have an optical property different from, for example, commercially available bacteria of the same species. It is believed that having such an optical property is involved in the exertion of antitumor activity.

The bacteria isolated from a tumor contained in the antitumor agent of the present invention may exhibit the following property regarding the absorption spectrum. The bacteria isolated from the tumor (particularly the purple non-sulfur bacteria) have a higher absorption property than normal bacteria of the same species (e.g., commercially available bacteria). It is considered that the higher absorption property are related to the antitumor activity of the bacteria (particularly the purple non-sulfur bacteria).

For example, when measuring the absorption spectrum of a dispersion liquid in which the bacterium contained in the antitumor agent (particularly the purple non-sulfur bacterium) is dispersed in a PBS buffer at a concentration of $2 \times 10^7$ CFU/mL, the absorbance at 808 nm is preferably 0.029 or more, more preferably 0.030 or more, and even more preferably 0.031 or more, 0.032 or more, 0.033 or more, 0.034 or more, or 0.035 or more. Such a high absorbance is considered to be associated with the antitumor activity of the bacterium (particularly the purple non-sulfur bacterium).

The upper limit of the absorbance at 808 nm is not particularly set, but may be, for example, 0.10 or less, 0.9 or less, or 0.8 or less.

The absorbance spectrum is measured as described in the Examples below.

In addition, when measuring the absorption spectrum of a dispersion liquid in which the bacterium contained in the antitumor agent (particularly the purple non-sulfur bacterium) is dispersed in a PBS buffer at a concentration of $2 \times 10^7$ CFU/mL, the absorbance at 865 nm is preferably 0.032 or more, more preferably 0.033 or more, and even more preferably 0.034 or more, 0.035 or more, 0.036 or more, 0.037 or more, or 0.038 or more. Such a high absorbance is considered to be associated with the antitumor activity of the bacterium (particularly the purple non-sulfur bacterium).

The upper limit of the absorbance at 865 nm is not particularly set, but may be, for example, 0.11 or less, 0.10 or less, or 0.09 or less.

Particularly preferably, when the absorption spectrum of the dispersion liquid is measured, the absorbance at 808 nm is preferably 0.029 or more, more preferably 0.030 or more, even more preferably 0.031 or more, 0.032 or more, 0.033 or more, 0.034 or more, or 0.035 or more, and the absorbance at 865 nm is preferably 0.032 or more, more preferably 0.033 or more, even more preferably 0.034 or more, 0.035 or more, 0.036 or more, 0.037 or more, or 0.038 or more. Such high absorbances at both of these two wavelengths are considered to be related to the bacterium (particularly the purple non-sulfur bacterium) having antitumor activity.

The bacteria isolated from a tumor contained in the antitumor agent of the present invention may exhibit the following property regarding the fluorescence spectrum. The bacteria isolated from the tumor (particularly the purple non-sulfur bacteria) have a higher fluorescence property than normal bacteria of the same species (e.g., commercially available bacteria). It is considered that the higher fluorescence property are related to the antitumor activity of the bacteria (particularly the purple non-sulfur bacteria).

For example, when measuring the fluorescence spectrum (excitation wavelength: 805 nm) of a dispersion liquid in which the bacterium contained in the antitumor agent is dispersed in a PBS buffer at a concentration of $2.5 \times 10^7$ CFU/mL, the fluorescence intensity at 888 nm is preferably 4.4 or more, more preferably 4.5 or more, and even more preferably 5.0 or more, 7.0 or more, or 9.0 or more.

The upper limit of the fluorescence intensity does not need to be particularly set, but may be, for example, 30 or less, 25 or less, or 20 or less.

Such high fluorescence intensity is believed to be associated with the antitumor activity of the bacteria (particularly the purple non-sulfur bacteria).

The fluorescence spectra are measured as described in the Examples below.

(Toxicity of Bacterium)

Preferably, the bacteria isolated from tumors contained in the antitumor agent of the present invention have no toxicity or, if any, very low toxicity, as described below. Unlike, for example, commercially available bacteria of the same species, the bacteria isolated from tumors may have such a toxicity property. The fact that the bacteria isolated from tumors have such no or very low toxicity property further enhances the usefulness of the antitumor agent.

For example, when the bacterium contained in the antitumor agent is administered once via the tail vein of a mouse in an amount of $1 \times 10^9$ CFU, the mouse survival rate for 40 days after administration may be preferably 90% or more, more preferably 95% or more, and may be, for example, 100%. The bacterium contained in the antitumor agent of the present invention may have such a toxicity property, particularly an extremely low toxicity property. Various conditions for determining the mouse survival rate (e.g., the medium in which the administered bacterium is suspended and the preparation method thereof, etc.) are as described in the Examples below.

(Immunostimulating property)

The bacterium isolated from the tumor and contained in the antitumor agent of the present invention may be a bacterium having an immunostimulating property. The antitumor agent may treat tumors by using the immunostimulating property.

The immunostimulating property may for example be a property that stimulates immune cells.

The immune cells include, for example, innate immune cells, and examples of the innate immune cells include macrophages, NK cells, and neutrophils. That is, the bacteria isolated from the tumor may have a property of activating innate immune cells, and may be, for example, a bacterium having a property of activating one, two, or all three of macrophages, NK cells, and neutrophils.

Furthermore, examples of the immune cells include acquired immune cells, and examples of the acquired immune cells include T cells and B cells. That is, the bacteria isolated from the tumor may have a property of activating acquired immune cells, and may be, for example, bacteria having a property of activating T cells, B cells, or both of these.

Such an immune cell activating property are believed to contribute to the antitumor activity of the bacteria isolated from the tumor.

The immunostimulating property may be, for example, a property of enhancing the expression of an antitumor marker. Examples of the antitumor marker include a necrosis marker and an apoptosis marker, and specific examples of these include TNF-$\alpha$ and caspase-3, respectively. That is, the bacterium isolated from the tumor may have a property of enhancing the expression of an antitumor marker, for example, a bacterium having a property of enhancing the expression of a necrosis marker or an apoptosis marker macrophage, or both of these.

Such an antitumor marker expression enhancing property is also believed to contribute to the antitumor activity of the bacteria isolated from the tumor.

In addition, since the bacteria isolated from the tumor have such an immunostimulating property, they may be used as an active ingredient of an immunostimulant. That is, the present invention also provides an immunostimulant containing the bacteria isolated from the tumor. The immunostimulant may be called an immunostimulating composition. The configuration of the antitumor agent described in this specification may be adopted as the configuration of the immunostimulant.

2.1.2 Antitumor agents containing bacteria of the genus Proteus isolated from tumors

In another embodiment of the present invention, the bacteria isolated from the tumor contained in the antitumor agent of the present invention include bacteria of the genus Proteus. As described in 1. above, the bacteria of the genus Proteus isolated from the tumor can exhibit excellent antitumor activity. Furthermore, since the bacteria of the genus Proteus gather in the tumor when administered to an animal, particularly when administered parenterally, they do not need to be administered directly to the tumor site, but may be administered directly to the tumor site. Furthermore, the bacteria of the genus Proteus may be administered for the treatment of tumors based on their antitumor activity.

In a preferred embodiment, the bacterium of the genus Proteus may be a bacterium purely isolated from a tumor. That is, the antitumor agent may contain only purely-isolated bacterium of the genus Proteus as the bacterium.

(Type of bacterium)

The bacterium of the genus Proteus may be, for example, any one, two, or three of Proteus mirabilis, Proteus vulgaris, and Proteus myxofaciens, and may preferably be Proteus mirabilis. Proteus mirabilis isolated (particularly purely isolated) from a tumor exhibits particularly excellent antitumor activity. The Proteus mirabilis may be, for example, a bacterium deposited under the accession number: NITE BP-03626. The bacterium was deposited internationally at National Institute of Technology and Evaluation, Patent Microorganisms Depositary (NPMD, Room 122, 2-5-8 Kazusa Kamatari, Kisarazu City, Chiba Prefecture, 292-0818) on March 23, 2022.

(Bacterial toxicity)

Preferably, the bacteria of the genus Proteus isolated from the tumor contained in the antitumor agent of the present invention have no toxicity or, if any, have very low toxicity, as described below. Unlike, for example, commercially available bacteria of the same species, the bacteria isolated from the tumor may have such a toxicity property. The fact that the bacteria isolated from the tumor have such no or very low toxicity property further enhances the usefulness of the antitumor agent.

For example, when the bacterium of the genus Proteus contained in the antitumor agent is administered once via the tail vein of a mouse in an amount of $1\times10^8$ CFU, the mouse survival rate for 40 days after administration may be preferably 90% or more, more preferably 95% or more, and may be, for example, 100%. The bacterium of the genus Proteus contained in the antitumor agent of the present invention may have such a toxicity property, particularly an extremely low toxicity property. Various conditions for determining the mouse survival rate (e.g., the medium in which the administered bacteria are suspended and the preparation method thereof, etc.) are as described in the Examples below.

(Immunostimulating property)

The bacterium of the genus Proteus isolated from the tumor may be a bacterium having an immunostimulating property. The antitumor agent may treat the tumor by using the immunostimulating property.

The immunostimulating property may for example be a property that stimulates immune cells.

The immune cells include, for example, innate immune cells, and examples of the innate immune cells include macrophages, NK cells, and neutrophils. That is, the bacteria of the genus Proteus isolated from the tumor may have a property of activating innate immune cells, and may be, for example, a bacterium having a property of activating one, two, or all three of macrophages, NK cells, and neutrophils.

Furthermore, examples of the immune cells include acquired immune cells, and examples of the acquired immune cells include T cells and B cells. That is, the bacteria of the genus Proteus isolated from the tumor may have a property of activating acquired immune cells, and may be, for example, a bacterium having a property of activating T cells, B cells, or both of these.

It is believed that such an immune cell activating property contribute to the antitumor activity of the bacteria of the genus Proteus isolated from the tumor.

The immunostimulating property may be, for example, a property of enhancing the expression of an antitumor marker. Examples of the antitumor marker include a necrosis marker and an apoptosis marker, and specific examples of these include TNF-$\alpha$ and caspase-3, respectively. That is, the bacterium of the genus Proteus isolated from the tumor may have a property of enhancing the expression of an antitumor marker, for example, a bacterium having a property of enhancing the expression of a necrosis marker or an apoptosis marker macrophage, or both of these.

Such a property of enhancing the expression of antitumor markers is also believed to contribute to exhibit the antitumor activity of the bacteria of the genus Proteus isolated from the tumor.

Since the bacteria of the genus Proteus isolated from the tumor have such an immunostimulating property, they may be used as an active ingredient of an immunostimulant. That is, the present invention also provides an immunostimulant containing the bacteria of the genus Proteus isolated from the tumor. The immunostimulant may be called an immunostimulating composition. The configuration of the antitumor agent described in this specification may be adopted as the configuration of the immunostimulant.

2.1.3 Other bacterial examples

In yet another embodiment of the invention, the bacteria isolated from the tumor may for example be other bacteria present within the tumor, in particular tumor-resident bacteria. Examples of such bacteria include bacteria belonging to the order Enterobacteriales, bacteria belonging to the order Pseudomonadales, bacteria belonging to the order Burkholderiales, bacteria belonging to the order Rhodobacterales, bacteria belonging to the order Aeromonadales, bacteria belonging to the order Bacillales, bacteria belonging to the order Clostridiales, bacteria belonging to the order Lactobacillales, bacteria belonging to the order Actinomycetales, bacteria belonging to the order Bifidobacteriales, bacteria belonging to the order Bacteroidales, bacteria belonging to the order Flavobacteriales, bacteria belonging to the order Fusobacteriales, bacteria belonging to the order Streptophyta, bacteria belonging to the order Corynebacteriales, bacteria belonging to the order Rhodospirillales, and bacteria belonging to the order Neisseriales. The bacteria used in the present invention may be any one type or a combination of two or more types of the bacteria belonging to these listed orders.
More specific examples of bacteria for each of the orders listed above include the following:

bacteria of the order Enterobacteriales: for example bacteria of the family Enterobacteriaceae, in particular bacteria of the genera Proteus, Enterobacter, Klebsiella and Citrobacter, more in particular the species of bacteria of the genera Proteus mentioned above in 2.1.2, as well as Enterobacter asburiae, Klebsiella pneumoniae, Citrobacter freundii and Enterobacter cloacae, etc.;
bacteria of the order Pseudomonadales: for example bacteria of the family Pseudomonadaceae and bacteria of the family Moraxellaceae, in particular bacteria of the genus Pseudomonas and bacteria of the genus Acinetobacter, more in particular Pseudomonas argentinensis and Acinetobacter US_424, etc.;
bacteria of the order Rhodobacterales: for example bacteria of the family Rhodobacteraceae, in particular bacteria of the genus Paracoccus, more particularly Paracoccus marcusii, etc.;
bacteria of the order Sphingomonadales: for example bacteria of the family Sphingomonadaceae, in particular bacteria of the genus Sphingomonas, more particularly Sphingomonas yunnanensis, Sphingomonas US_602 and Sphingomonas yanoikuyae, etc.;
bacteria of the order Bacillales: for example bacteria of the family Staphylococcaceae, in particular bacteria of the genus Staphylococcus, more in particular Staphylococcus aureus and Staphylococcus cohnii, etc.;
bacteria of the order Lactobacillales: for example bacteria of the family Streptococcaceae and bacteria of the family Lactobacillaceae, in particular bacteria of the genus Streptococcus and bacteria of the genus Lactobacillus, more in particular Streptococcus infantis and Lactobacillus iners, etc.;
bacteria of the order Actinomycetales: for example, bacteria of the family Actinomycetaceae, in particular bacteria of the genus Actinomyces, more in particular Actinomyces massiliensis, etc.;
bacteria of the order Fusobacteriales: for example bacteria of the family Fusobacteriaceae, in particular bacteria of the genus Fusobacterium, more in particular Fusobacterium nucleatum, etc.;
bacteria of the order Corynebacteriales: for example bacteria of the family Corynebacteriaceae, in particular bacteria of the genus Corynebacterium, more in particular Corynebacterium US_1715, etc.;
bacteria of the order Rhodospirillales: for example bacteria from the family Acetobacteraceae, in particular bacteria of the genus Roseomonas, more in particular Roseomonas mucosa, etc.; and
bacteria of the order Neisseriales: for example bacteria of the family Neisseriaceae, in particular bacteria of the genus Neisseria, more in particular Neisseria macacae, etc.

In the present invention, any one type or a combination of two or more types of the bacteria belonging to the above-listed families or genera may be isolated from a tumor. That is, in the present invention, the one type or a combination of two or more types of the bacteria isolated from a tumor may be used in the present invention.

2.1.4 Composition of antitumor agent

The antitumor agent according to the present invention may contain the bacteria isolated from the tumor in a viable state. That is, the bacteria isolated from the tumor may be administered to an animal in a viable state. This is believed to facilitate the bacteria's delivery to the tumor site, and also to allow the bacteria's antitumor activity to be more effectively exerted. Delivery to the tumor site is also useful for diagnosis, which will be described later.
The antitumor agent according to the present invention may contain the bacteria isolated from the tumor in a dead state.

In the present invention, the term "antitumor agent" may refer to an agent, particularly a drug, used to treat a tumor occurring in an animal. "Tumor treatment" may refer to, for example, one or more of reducing the size of a tumor, inhibiting tumor growth, killing or reducing tumor cells, or inhibiting tumor cell proliferation.

An "antitumor agent" may refer to an agent used to treat or prevent an animal having a tumor. The animal may be, for example, a mammal, particularly a human, but may also be a non-human animal. The non-human animal may be, for example, a farm animal or a pet animal, such as a cow, horse, sheep, goat, pig, dog, cat, or rabbit.

The "agent" may be an agent consisting of one type of component, but may also be an agent containing two or more components. At least one of these components is the bacterium isolated from the tumor described above. Since the agent may contain two or more components, in this case, the antitumor agent of the present invention may be called an antitumor composition, or even an antitumor pharmaceutical composition.

That is, the antitumor agent of the present invention may contain other components in addition to the bacteria isolated from a tumor. The other components may be appropriately selected by those skilled in the art depending on factors such as the method or site of administration of the antitumor agent.

The antitumor agent of the present invention may be used to treat malignant or benign tumors, and is particularly preferably used to treat malignant tumors. The malignant tumor is also called "cancer". Cancer can be classified into solid cancer and blood cancer. Solid cancer can be further classified into carcinoma and sarcoma. The antitumor agent may be called an anticancer agent, or a composition for cancer treatment or a composition for cancer therapy.

The antitumor agents of the present invention may be used to treat solid or hematological cancers, in particular to treat solid cancers, for example to treat carcinomas or to treat sarcomas.

The antitumor agent of the present invention can be used to treat various cancers. One of the mechanisms of the antitumor activity of the antitumor agent of the present invention is thought to be the stimulation of immune cells, as described below. In other words, the antitumor agent is thought to exert its antitumor activity via the activated immune cells. Therefore, the antitumor agent of the present invention is thought to be effective against not only one specific type of cancer, but also various cancers, and its effectiveness against various cancers is actually shown in the examples described below.

In one embodiment, the antitumor agent of the present invention may be used to treat carcinoma. The antitumor agent of the present invention may be an agent used for treating, for example, any one or more of head and neck cancer (e.g., pharyngeal cancer, laryngeal cancer, tongue cancer, etc.), esophageal cancer, gastric cancer, duodenal cancer, colorectal cancer (e.g., colon cancer, rectal cancer, etc.), liver cancer, gallbladder cancer, bile duct cancer, pancreatic cancer, anal cancer, kidney cancer, bladder cancer, prostate cancer, uterine cancer (e.g., cervical cancer, uterine body cancer), and ovarian cancer.

In another embodiment, the antitumor agent of the present invention may be used to treat sarcoma. The antitumor agent of the present invention may be an agent used for treating, for example, any one of osteosarcoma, chondrosarcoma, rhabdomyosarcoma, leiomyosarcoma, fibrosarcoma, liposarcoma, and angiosarcoma.

In one embodiment, the antitumor agent of the present invention may be configured as a liquid agent. That is, the liquid agent may be a liquid agent containing the bacteria isolated from a tumor according to the present invention (particularly a dispersion liquid of the bacteria). The liquid component other than the bacteria in the liquid agent may be, for example, an injection solution or drip infusion solution used in the pharmaceutical field, and more specifically, may be an isotonic solution, a hypotonic solution, or a hypertonic solution. For example, the liquid may be saline (physiological saline), a sugar solution, or a buffer solution. The liquid may also be phosphate buffered saline. The dosage form of a liquid agent is particularly suitable for allowing the antitumor agent of the present invention to reach the tumor while maintaining its antitumor activity.

The antitumor agent of the present invention is preferably administered parenterally. For example, the antitumor agent of the present invention may be administered intravascularly (e.g., intravenously or intraarterially), subcutaneously, intramuscularly, or intrathecally. Preferably, the antitumor agent of the present invention is administered intravascularly (e.g., intravenously or intraarterially).

The bacteria isolated from tumors contained in the antitumor agent of the present invention have the property of gathering at tumor sites as described above, so that the bacteria can easily reach tumor sites by administering the agent into blood vessels. Intravascular administration is also relatively less invasive.

Furthermore, when bacteria are administered into a blood vessel, in many cases the bacteria exert toxicity and adversely affect the patient. However, as described above, the bacteria isolated from tumors contained in the antitumor agent of the present invention do not exert such toxicity, or if they do, the degree of toxicity is very low.

The antitumor agent of the present invention may also be administered directly to the tumor site or near the tumor site, for example, using a syringe or other tube, etc. The antitumor agent of the present invention may also be administered orally, depending on the location of the tumor.

The antitumor agent of the present invention may be administered so that the bacteria isolated from a tumor are at, for example, $10^4$ CFU/kg body weight up to $10^{11}$ CFU/kg body weight, preferably $10^5$ CFU/kg body weight up to $10^{11}$

CFU/kg body weight, more preferably $10^6$ CFU/kg body weight up to $10^{11}$ CFU/kg body weight, per one administration. If the amount of bacteria administered is too small, the antitumor effect may be reduced. Also, if the amount of bacteria administered is too large, it is undesirable from the viewpoint of cost.

When the antitumor agent of the present invention is a liquid agent, the antitumor agent may contain, for example, the bacteria isolated from a tumor at a concentration of, for example, $10^5$ CFU/ml up to $10^{12}$ CFU/ml, preferably $10^6$ CFU/ml up to $10^{12}$ CFU/ml, more preferably $10^7$ CFU/ml up to $10^{12}$ CFU/ml.

The antitumor agent of the present invention may be administered only once or twice or more. The antitumor agent of the present invention can exert its effect by a single administration, but may be administered twice or more as necessary.

The antitumor agent of the present invention may be administered once per day, or may be administered two or more times (e.g., two or three times) per day.

When the antitumor agent of the present invention is administered multiple times, the antitumor agent may be administered every day, every other day, or every third day. Further, the antitumor agent may be administered every week, every two weeks, every three weeks, or every four weeks.

The antitumor agent of the present invention may contain additives used in formulation in the pharmaceutical field, for example, various components such as pH adjusters and colorants. In addition, the antitumor agent of the present invention may contain known or future medicinal components for tumor treatment, so long as they do not impair the effects of the present invention. The formulation of the antitumor agent of the present invention may be carried out by a known method as appropriate depending on the dosage form.

The antitumor agent of the present invention may be used not only for the treatment of tumors as described above, but also for the diagnosis of tumors. As described below, the bacteria isolated from the tumor have the property of gathering in tumors when administered. Therefore, by utilizing this property, the site where the bacteria gather can be identified as a tumor. Please refer to the explanation below for the diagnosis.

2.1.5 Method for producing antitumor agent

The bacterium described above is used to produce the antitumor agent. The bacterium may be produced, for example, as follows.

(Tumor collection step S11)

In the tumor collection step, the tumor is collected from the tumor-bearing animal. The collection may be performed, for example, by biopsy. The tumor may be, for example, a tumor formed from mammalian tumor cells, in particular a tumor formed from human tumor cells. The tumor-bearing animal may be, for example, a mammal, in particular a rodent, but may also be a primate, in particular a human.

The tumor-bearing animal may be administered with a purple non-sulfur bacterium prior to the collection. The administered purple non-sulfur bacterium may be any of the purple non-sulfur bacteria listed in 2.1.1 above, preferably bacteria of the genus Rhodopseudomonas or bacteria of the genus Blastochloris, and more preferably bacteria of the genus Rhodopseudomonas. Specific species of these bacteria are as described in 2.1.1 above, and any one or more species of bacteria may be administered. The administered bacteria may be commercially available.

When the purple non-sulfur bacteria are administered as described above, the administration is, for example, parenterally, particularly intravenously. After a predetermined period of time, for example, 1 to 10 days, particularly 2 to 5 days, following the administration, the tumor is collected as described above. It is believed that the administered purple non-sulfur bacteria reach the tumor by the passage of such a period of time.

The bacterial administration may not be performed, but may be performed. When the bacterial administration is not performed, bacteria originally present in the tumor may be collected. When the bacterial administration is performed, bacteria originally present in the tumor may be collected, or bacteria administered by the bacterial administration and delivered to the tumor may be collected.

(Bacteria isolation step S12)

In the bacteria isolation step, bacteria are isolated from the tumor collected in the tumor collection step. For example, the tumor obtained by biopsy is added to a specific liquid (e.g., a buffer solution, etc.) and homogenized. The homogenization produces a liquid in which the tumor cells and bacteria are suspended. By shaking the liquid, for example at a certain speed, the tumor cells are precipitated and the bacteria are present in the supernatant. In this manner, the bacteria may be isolated from the tumor cells, but the method of isolation is not limited thereto. The bacteria may also be isolated by other methods known in the art.

The presence of the bacteria in tumors and their isolation from tumors are believed to contribute to the bacteria's antitumor activity.

(Bacterial culture step S13)

In the bacteria culturing step, the bacteria isolated from the tumor in the bacteria isolating step are cultured. Cultivation of the bacteria isolated from the tumor is also considered to contribute to the bacteria acquiring antitumor activity.

For example, in the bacterial culture step, the supernatant may be added onto a general-purpose agar medium in a petri dish, and cultured. This allows colonies to form on the medium. The colonies may be used as bacteria isolated from tumors in the present invention.

The general-purpose agar medium may be, for example, a peptone-containing medium or a peptone-free medium.

The peptone contained in the peptone-containing medium may be, for example, one or more of casein peptone, meat peptone, gelatin peptone, and soybean peptone. The peptone-containing medium may be, for example, LB medium or polypeptone medium. In addition to the peptone, the peptone-containing medium may further contain an extract. The extract may be, for example, yeast extract or meat extract (particularly beef extract), or a combination thereof.

The peptone-free medium may, for example, comprise an extract. The extract may be yeast extract or meat extract (particularly beef extract) or a combination thereof, as described above. The peptone-free medium may, for example, be ATCC 543 medium.

The general-purpose agar medium may be appropriately selected by those skilled in the art depending on the bacterium to be isolated.

Preferably, the colonies (particularly bacteria in the colonies) formed on the agar medium are further cultured in a liquid medium.

The liquid medium may be a liquid general-purpose medium. The liquid general-purpose medium may be, for example, a peptone-containing medium as described above, or may be a peptone-free medium. The peptone and extract contained in these media are as described above, and the same description also applies to the liquid medium. The liquid medium may be appropriately selected by a person skilled in the art depending on the bacterium to be isolated.

(Culture in medium without cysteine)

In one embodiment, the liquid medium may be a liquid medium to which cysteine is not added. The liquid medium may be, for example, a liquid medium to which cysteine is not added, which contains peptone, and which contains an extract (particularly yeast extract). The liquid medium may be, for example, an LB medium or a polypeptone medium to which cysteine is not added. Alternatively, the liquid medium may be a liquid medium to which cysteine is not added, which does not contain peptone, and which contains an extract (particularly yeast extract). An example of such a medium may be an ATCC 543 medium to which cysteine is not added.

The bacteria cultured in the liquid medium may be further cultured on a general-purpose agar medium to form colonies. The general-purpose agar medium may be, for example, a general-purpose agar medium to which deoxycholic acid has been added. The ratio (%) of the content (g) of deoxycholic acid in the agar medium to the amount (g) of the agar medium may be, for example, 0.01% to 1%, preferably 0.03% to 0.5%, and more preferably 0.05% to 0.3%. The general-purpose agar medium may be the same agar medium (fresh medium) as the general-purpose agar medium described at the beginning of this step, except that deoxycholic acid has been added. Colonies are formed on the medium by the culture.

The formed colonies may be further cultured in a liquid medium. The liquid medium may be the same medium (fresh medium) as that used in the previous liquid culture.

As described above, the bacteria cultured in a medium to which cysteine has not been added may be used as an active ingredient of the antitumor agent of the present invention.

This embodiment is suitable for obtaining, for example, a purple non-sulfur bacterium (particularly a bacterium of the genus Rhodopseudomonas) having antitumor activity or a composite bacterium of a purple non-sulfur bacterium (particularly a bacterium of the genus Rhodopseudomonas) having antitumor activity and another bacterium (particularly a bacterium of the genus Proteus).

(Culture in medium supplemented with cysteine)

In another embodiment, the liquid medium may be a liquid to which cysteine is added. The liquid medium may be, for example, a liquid medium to which cysteine is added, containing peptone and containing an extract (particularly yeast extract). The liquid medium may be, for example, an LB medium or a polypeptone medium to which cysteine is added. Alternatively, the liquid medium may be a liquid medium to which cysteine is added, not containing peptone and containing an extract (particularly yeast extract). An example of such a medium may be an ATCC 543 medium supplemented with cysteine.

The cysteine content in the liquid medium to which cysteine has been added, when expressed as a ratio (%) of the amount (g) of the cysteine to the amount (g) of the medium, may be, for example, 0.1% to 10%, more preferably 1% to 5%, and even more preferably 2% to 4%.

The bacteria cultured in the liquid medium may be further cultured on a general-purpose agar medium to form colonies. The general-purpose agar medium may be, for example, a general-purpose agar medium to which deoxycholic acid and cysteine have been added. The ratio (%) of the content (g) of deoxycholic acid in the agar medium to the amount (g) of the agar medium may be, for example, 0.01% to 1%, preferably 0.03% to 0.5%, and more preferably 0.05% to 0.3%. The ratio (%) of the content (g) of cysteine in the agar medium to the amount (g) of the agar medium may be, for example, 0.1% to 10%, more preferably 1% to 5%, and even more preferably 2% to 4%. The general-purpose agar medium may be the same agar medium (fresh medium) as the general-purpose agar medium described at the beginning of this step, except that deoxycholic acid and cysteine have been added. Colonies are formed on the medium by the culture.

The formed colonies may be further cultured in a liquid medium. The liquid medium may be the same medium (fresh medium) as that used in the previous liquid culture.

The bacteria cultured as described above may be used as an active ingredient of the antitumor agent of the present invention.

This embodiment is suitable, for example, for obtaining bacteria of the genus Proteus with antitumor activity.

(Preparation step S14)

The bacteria cultured in the bacterial culture step are used to prepare a formulation to obtain an antitumor agent. The antitumor agent may be, for example, the liquid agent described above. The liquid agent may be produced by mixing the bacteria with a predetermined liquid. The method of preparation may be appropriately selected by those skilled in the art based on, for example, the dosage form or the ingredients contained therein.

2.2 Second embodiment (bacteria)

The present invention also provides a bacterium isolated from a tumor. The bacterium has antitumor activity as described above. Therefore, it may be used, for example, to manufacture an antitumor agent. In addition, since the bacterium has the property of gathering in tumors as described above, it may be used to generate an image for identifying a tumor, and may be used, for example, to manufacture a diagnostic drug.

2.2.1 Purple non-sulfur bacteria

In one embodiment of the present invention, the bacteria isolated from the tumor comprise at least one or more species of purple non-sulfur bacteria isolated from the tumor. The purple non-sulfur bacteria are as described in 2.1.1 above, and the description therein (e.g., type of bacteria, optical property of bacteria, toxicity of bacteria) also applies to this embodiment.

In one embodiment, the bacteria isolated from the tumor comprise a bacterium of the genus Rhodopseudomonas or a bacterium of the genus Blastochloris isolated from the tumor, or both, and particularly preferably comprise a bacterium of the genus Rhodopseudomonas isolated from the tumor. The species of bacteria belonging to these genera may be as described in 2.1.1 above.

The bacteria isolated from the tumor may contain, in addition to the purple non-sulfur bacteria, other bacteria isolated from the tumor. The other bacteria may be, for example, bacteria of the genus Proteus. That is, the bacteria isolated from the tumor may be a combination of the purple non-sulfur bacteria and other bacteria, for example, a combination of the purple non-sulfur bacteria (particularly bacteria of the genus Rhodopseudomonas) and the bacteria of the genus Proteus. As described in 2.1.1 above, this combination exhibits particularly excellent antitumor activity.

2.2.2 Bacteria of the genus Proteus

In another embodiment of the present invention, the bacteria isolated from the tumor comprise at least one species of bacterium of the genus Proteus isolated from the tumor. The bacterium of the genus Proteus is as described in 2.1.2 above, and the description thereof (e.g., type of bacteria and bacterial toxicity) also applies to this embodiment.

2.3 Third embodiment (image generation method)

The present invention also provides an image generation method. The method may include, for example, an administration step of administering bacteria isolated from a tumor to an animal, and an image generation step of imaging the animal after the administration while irradiating light in a predetermined wavelength range to generate an image for identifying a tumor that may be present in the animal. The bacteria isolated from a tumor according to the present invention have a specific optical property and also have a property of congregating at the tumor site when administered to an animal. Thus, by utilizing these properties, it is possible to determine the presence or absence of a tumor or to identify the position or shape of the tumor. Note that the image generated in this manner may be used not only to identify the tumor, but also to identify the location where the administered bacteria are concentrated.

Each step involved in the method is described below.

(Administration step S21)

In the administration step, bacteria isolated from a tumor are administered to an animal. The bacteria isolated from the tumor preferably include purple non-sulfur bacteria. The purple non-sulfur bacteria are as described in 2.1.1 above, and the description also applies to this embodiment. In addition to the purple non-sulfur bacteria, the bacteria may include other bacteria (particularly bacteria of the genus Proteus) as described in 2.1.1 above. The purple non-sulfur bacteria preferably have the optical property described in 2.1.1 above. The purple non-sulfur bacteria preferably have the toxic property described in 2.1.1 above.

In the administration step, the bacteria isolated from the tumor are preferably administered parenterally. For example, the bacteria may be administered intravascularly (e.g., intravenously or intraarterially), subcutaneously, intramuscularly, or intrathecally. Preferably, the bacteria are administered intravascularly (e.g., intravenously or intraarterially), particularly intravenously.

The bacteria isolated from tumors contained in the antitumor agent of the present invention have the property of gathering at tumor sites as described above, so that the bacteria can easily reach tumor sites by administering the agent into blood vessels. Intravascular administration is also relatively less invasive.

Furthermore, when bacteria are administered into a blood vessel, the bacteria usually exert toxicity and have adverse effects on the patient. However, as described above, the bacteria isolated from tumors contained in the antitumor agent of the present invention do not exert such toxicity, or if they do, the degree of toxicity is very low.

For parenteral administration, the bacteria may be formulated as a liquid agent. The liquid agent may be formulated in the same manner as the liquid agent described in 2.1.4 above, and the same explanation applies to this embodiment. The amount of bacteria contained in the liquid agent may also be the same as the bacterial content in the liquid agent described in 2.1.4 above.

(Imaging step S22)

After the administering step, the animal is imaged while illuminated with light of a predetermined wavelength range. By this imaging, an image for identifying possible tumors in the animal is generated. The wavelength range of the light may be selected according to the optical property of the administered bacteria.

The light may be, for example, infrared light, and may be, in particular, near-infrared light, mid-infrared light, or far-infrared light. The infrared light has little effect on the irradiated animal. Furthermore, the purple non-sulfur bacteria isolated from the tumor emit fluorescence when irradiated with infrared light (in particular, near-infrared light), and the fluorescence intensity of the fluorescence is strong. Therefore, an image obtained by imaging the animal while irradiating it with infrared light is suitable for identifying the tumor.

For the imaging, a device capable of observing the fluorescence generated from the bacteria may be used. The device may be, for example, a device capable of acquiring a fluorescent image of an imaging target, and more specific examples of the device include, but are not limited to, a fluorescent imaging system, an in vivo fluorescent imaging system, and a fluorescent imager.

The image obtained by the imaging makes it possible to identify, for example, the site where the fluorescence originating from the bacteria is generated.

The bacteria have the property of concentrating in tumors. Thus, if a fluorescent site is present in the image, the site may be identified as having a tumor. Also, the position or shape of the tumor may be identified based on the image.

Furthermore, if no fluorescent site is present in the image, it may be determined that no tumor is present in the imaging subject.

In this manner, the images obtained by the imaging are useful for identifying the presence or absence of a tumor, or identifying the location or shape of a tumor.

2.4 Fourth embodiment (bacterium production method)

The present invention also provides a method for producing useful bacteria. For example, in one embodiment, the bacteria produced by the production method may be bacteria having antitumor activity. In addition, the bacteria produced by the production method may be bacteria having the optical property described in 2.1.1 above.

The production method of the present invention may include, for example, a bacterial isolation step of isolating bacteria from a tumor, and a bacterial culture step of culturing the bacteria isolated in the isolation step. As described in 1. above, bacteria present in a tumor are isolated from the tumor and cultured to acquire antitumor activity or an optical property, or both. The bacterial isolation step and the bacterial culture step are as described in 2.1.5 above, and the description also applies to this embodiment.

The production method may further include a tumor collection step of collecting the tumor containing the bacteria, which is performed before the bacterial isolation step. The tumor collection step is also as described in 2.1.5 above, and the description thereof also applies to this embodiment.

2.5 Fifth embodiment (treatment method and diagnosis method)

The present invention provides a method for treating a tumor using a bacterium isolated from a tumor. In the treatment method, the bacterium isolated from the tumor may be used as described for the antitumor agent in 2.1 above. That is, the treatment method may include administering the antitumor agent to an animal. The administration may also be carried out as described in 2.1 above.

The present invention also provides a method for diagnosing a tumor using a bacterium isolated from a tumor. In the diagnostic method, the bacterium may be used as described in the image generation method in 2.3 above. Then, a diagnosis of the tumor may be made based on an image obtained in the image generation method.

The bacteria may also be used to both treat and diagnose a tumor. That is, the present invention also provides a method for treating and diagnosing a tumor using bacteria isolated from a tumor. In the method, the bacteria are administered to an animal (particularly a human) for treating a tumor. The administered bacteria reach the tumor, exert the antitumor activity thereof, and are used for an image generation method based on the optical property thereof. That is, in the method, the bacteria may be administered for treating a tumor, and the image generation method may be performed after the administration. Then, a tumor may be diagnosed based on an image obtained in the image generation method.

Examples

3. Example

<Cell culture and cytotoxicity evaluation>

Mouse colon cancer cells (Colon26) were obtained from the Japanese Collection of Research Bioresources Cell Bank. Mouse sarcoma cells (Meth-A) and mouse melanoma cells (B16-F10) were obtained from the Institute of Development, Aging and Cancer, Tohoku University (Cell Resource Center for Biomedical Research, Cell Bank). Roswell Park Memorial Institute (RPMI) 1640 Medium (Gibco) containing 10% fetal bovine serum, 2-mM l-glutamine, 1-mM sodium pyruvate, gentamycin, and penicillin-streptomycin (100 IU ml-1) was used to culture Colon26, Meth-A, and B16-F10. The cells were cultured in a humidified chamber at 37°C under a 5% $CO_2$ atmosphere.

<Bacterial Culture>

The bacteria used in this example were obtained from the National Institute of Technology and Evaluation Biological Resource Center (NBRC). Rhodopseudomonas Palustris (hereinafter also referred to as "RP") (NBRC 16661) was anaerobically cultured in 543 ATCC medium at a temperature of 26-30°C while irradiating with a tungsten lamp. Proteus mirabilis (hereinafter also referred to as "PM") (NBRC 3849) was anaerobically cultured in 802 NBRC medium at a temperature of 30°C. Reagents used for bacterial culture were obtained from FUJIFIILM Wako Pure Chemical, Nacalai Tesque, Tokyo Chemical Industry.

<Isolation and culture of intratumoral bacteria>

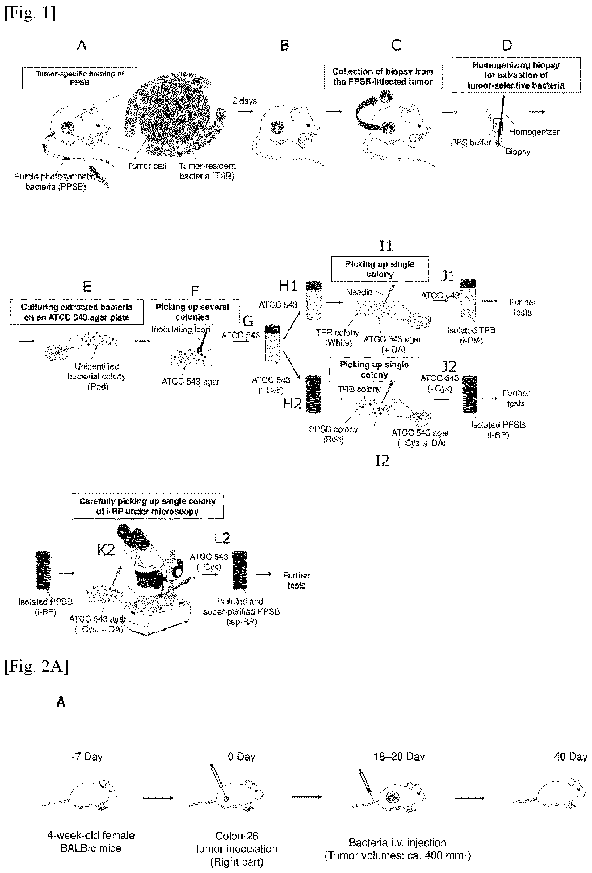

The isolation and culture of intratumoral bacteria are described with reference to Fig. 1.

All animal experiments were performed with the approval of the Animal Experiment Committee of the Japan Advanced Institute of Science and Technology. Four-week-old female wild-type mice (n=10; average weight= 15 g; BALB/cCrSlc) were purchased from Japan SLC, Inc. and allowed to acclimate for one week. A mixture of Colon26 cell dispersion liquid ($1\times10^6$ cells) (100 μL) and culture medium/matrigel (Corning) (v/v, 1: 1) was administered subcutaneously at one site on the right flank of the mice to create colon cancer model mice. Approximately two weeks later, RP ($5\times10^9$ CFU/mL) was administered at 200 μL into the tail vein of the mice bearing a solid tumor (up to 400 mm$^3$) (A in Fig. 1). Two days after administration, the presence of RP in the tumor (near-infrared fluorescence derived from RP) was confirmed using VISQUE (trademark) In Vivo Smart-LF (Vieworks) (B in Fig. 1), and the tumor was excised (C in Fig. 1). The tumor was cut into small pieces with a scalpel and then homogenized in a PBS buffer at 4°C using a pestle (D in Fig. 1). The resulting mixture was shaken at 380 rpm/min for 20 minutes at 15°C. After the shaking, the supernatant of the mixture was diluted 10-fold with a PBS buffer to obtain a bacterial sample.

The sample (100 μL) was inoculated onto an agar medium consisting of 543 ATCC medium and cultured anaerobically for 7 days (E in Fig. 1). Bacterial colonies formed on the agar medium during the anaerobic culture. Several red bacterial colonies were picked up with a loop (F in Fig. 1) and cultured anaerobically at 26-30°C in 543 ATCC medium while irradiating it with a tungsten lamp (G in Fig. 1). The culture solution obtained from the anaerobic culture was divided into two and used for the next culture.

Then, one of the cultures was cultured in normal 543 ATCC medium supplemented with cysteine (Cys) (H1 in Fig. 1; the culture obtained by this culture is hereinafter also referred to as "culture 1"). The cysteine content was 3% when expressed as the ratio (%) of the amount of cysteine (g) to the amount of 543 ATCC medium (g). The cysteine content in other cysteine-supplemented media described below was also 3%, regardless of whether the medium was a liquid medium or an agar medium.

The other was cultured in 543 ATCC medium without Cys (H2 in Fig. 1; the culture obtained by this culture is hereinafter also referred to as "culture 2").

Culture 1 was anaerobically cultured at 26-30°C on an agar medium consisting of 543 ATCC medium supplemented with Cys and 0.1% deoxycholic acid while irradiating with a tungsten lamp (I1 in Fig. 1). The deoxycholic acid content is the ratio (%) of the amount (g) of the deoxycholic acid to the amount (g) of the agar medium. The deoxycholic acid content in the other deoxycholic acid-supplemented media mentioned below, including the agar medium used for the culture of Culture 2, was 0.1%.

Culture 2 was anaerobically cultured at 26-30°C under tungsten lamp irradiation on an agar medium consisting of 543 ATCC medium without Cys supplemented with 0.1% deoxycholic acid (I2 in Fig. 1).

One white colony on the agar medium consisting of 543 ATCC medium was picked up with a needle (I1 in Fig. 1) and then cultured in 543 ATCC medium (J1 in Fig. 1). In this way, bacteria isolated from the tumor were obtained. Genetic identification of the bacteria (Bex Co., Ltd.) revealed that the bacteria were Proteus mirabilis. The isolated Proteus mirabilis is also referred to as " i-P.M." in this specification.

The Proteus mirabilis was deposited internationally on March 23, 2022, under the accession number NITE BP-03626 at National Institute of Technology and Evaluation, Patent Microorganism Depositary (NPMD) (Room 122, 2-5-8 Kazusa Kamatari, Kisarazu City, Chiba Prefecture, 292-0818).

A single red colony on an agar medium consisting of 543 ATCC medium without Cys was picked up with a needle (I2 in Fig. 1) and subsequently cultured in 543 ATCC medium without Cys (J2 in Fig. 1). In this way, bacteria isolated from the tumor were obtained. The bacteria contained two species, Rhodopseudomonas Palustris and Proteus mirabilis, that is, a complex bacterium. The composition ratio of Rhodopseudomonas Palustris and Proteus mirabilis in the complex bacterium was 97:3 based on CFU. The complex bacterium is also referred to as " i-R.P." in this specification.

The complex bacteria were deposited internationally at National Institute of Technology and Evaluation, Patent Microorganism Depositary (NPMD, Room 122, 2-5-8 Kazusa Kamatari, Kisarazu City, Chiba Prefecture, 292-0818) under the deposit number NITE BP-03627 on March 23, 2022.

i-R.P. was further cultured on an agar medium consisting of 543 ATCC medium without Cys and with 0.1% deoxycholic acid. From among the colonies that appeared on the agar medium, red colonies were carefully picked up with a needle under a microscope (K2 in Fig. 1), and were subsequently cultured in 543 ATCC medium without Cys (L2 in Fig. 1). In this way, a bacterium purely-isolated only to Rhodopseudomonas Palustris was obtained from the complex bacteria. This bacterium is also referred to as "isp-RP" in this specification.

<In vivo anti-cancer experiment>

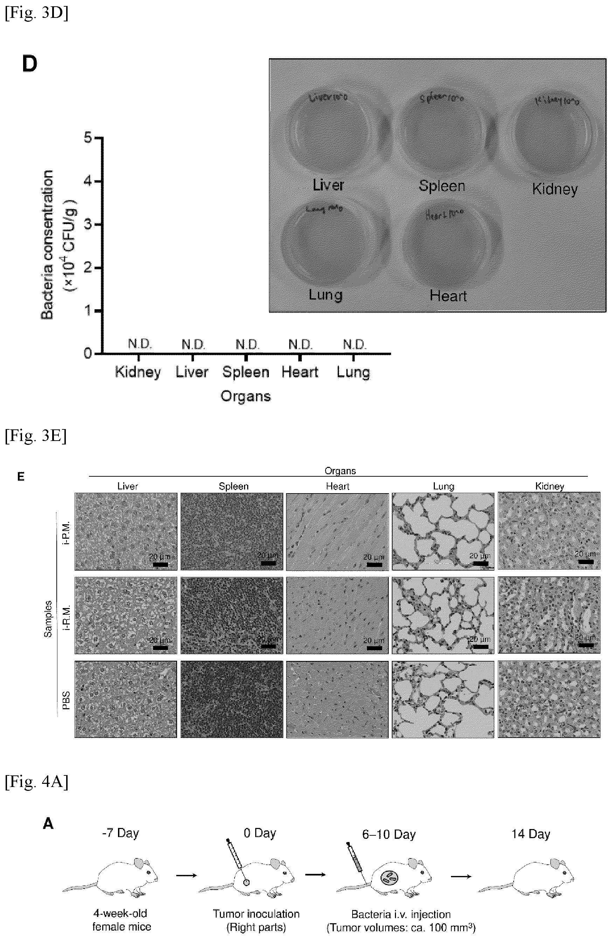

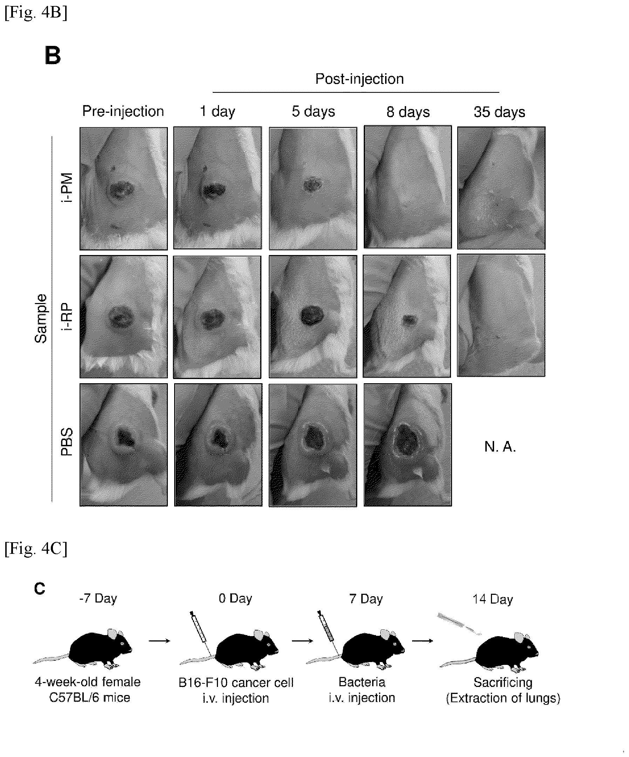

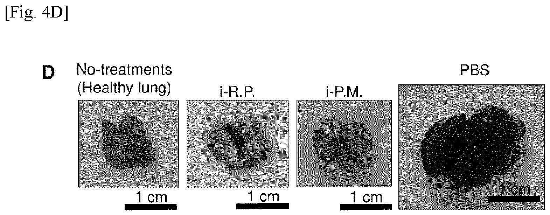

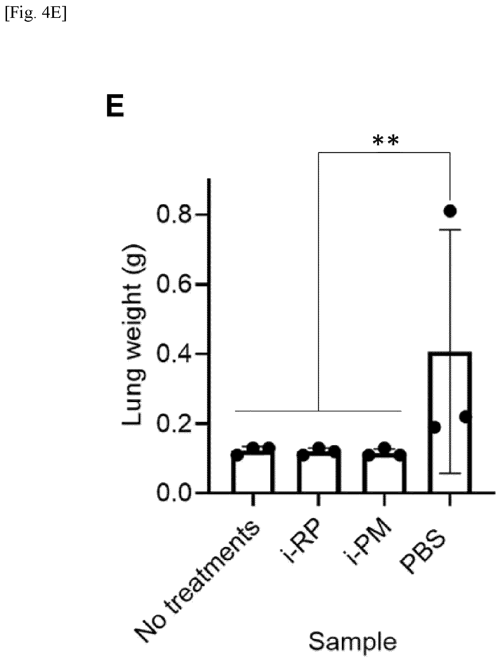

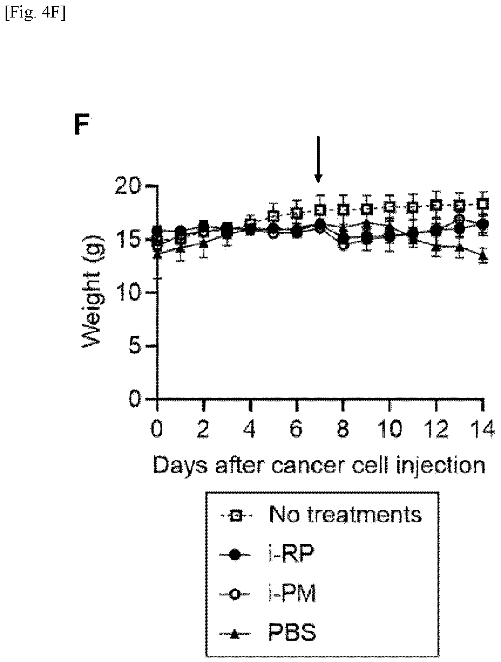

As shown in Fig. 2A, 4-week-old female wild-type mice (n=4; average weight= 15 g; BALB/cCrSlc) were purchased from Japan SLC, Inc. and allowed to acclimate for one week. A mixture of Colon26 cell dispersion liquid ($1\times10^6$ cells) (100 μL) and culture medium/matrigel (Corning) (v/v, 1:1) was administered subcutaneously at one site on the flank of the mice to create colon cancer model mice. Approximately two weeks later, 200 μL of bacterial sample of RP ($1\times10^9$ CFU), PM ($1\times10^8$ CFU), i-R.P.($1\times10^9$ CFU), or i-P.M. ($1\times10^8$ CFU) or PBS buffer solution was administered (single dose) into the tail vein of the mice bearing a solid tumor (up to 400 mm³). The behavior and property of the mice, the size measurement of the solid tumor, and the change in mouse weight were monitored daily. The size of the solid tumor was calculated using the following formula.

$$V = L \times W^2/2$$

In this formula, V is the volume of the solid tumor, L is the length of the solid tumor, and W is the width of the solid tumor.

The methods for preparing the above-mentioned four bacterial samples administered to the mice are described below.

i-R.P. was anaerobically cultured in Cys-free ATCC543 medium for 3 days at 26°C-30°C while irradiating with a tungsten lamp. The culture solution obtained by the anaerobic culture was centrifuged at 3000 rpm for 5 minutes. The bacteria precipitated by the centrifugation were suspended in fresh Cys-free ATCC543 medium to obtain a suspension. The number of bacteria in the suspension was measured using a microorganism counter (Panasonic, model number NP-BCM01-A), and based on the measurement result, fresh Cys-free ATCC543 medium was appropriately added to the suspension to obtain a predetermined bacterial concentration, thereby obtaining a bacterial sample of i-R.P.

RP was anaerobically cultured in ATCC543 medium supplemented with Cys for 3 days at 26°C-30°C while irradiating with a tungsten lamp. The culture solution obtained by the anaerobic culture was centrifuged at 3000 rpm for 5 minutes. The bacteria precipitated by the centrifugation were suspended in a fresh ATCC543 medium supplemented with Cys to obtain a suspension. The number of bacteria in the suspension was measured using a microorganism counter (Panasonic, model number NP-BCM01-A), and based on the measurement result, fresh ATCC543 medium supplemented with Cys was appropriately added to the suspension to obtain a predetermined bacterial concentration, thereby obtaining a bacterial sample of RP.

The bacterial samples of i-P.M. were obtained by the same method as the bacterial samples of RP. That is, by anaerobic cultivation in ATCC543 medium supplemented with Cys, centrifuging the culture obtained by the anaerobic cultivation, adjusting a suspension using the precipitate obtained by the centrifugation, and adjusting the bacterial concentration in the suspension.

P.M. was anaerobically cultured in 802 NBRC medium at 30°C for 3 days. The culture solution obtained by the anaerobic culture was centrifuged at 3000 rpm for 5 minutes. The bacteria precipitated by the centrifugation were suspended in fresh 802 NBRC medium to obtain a suspension. The number of bacteria in the suspension was measured using a microorganism counter (Panasonic, model number NP-BCM01-A), and based on the measurement results, fresh 802 NBRC medium was appropriately added to the suspension to obtain a predetermined bacterial concentration, and a bacterial sample of PM was obtained.

The CFU value described above for each bacterial sample is the CFU value of the bacteria in 200 $\mu$L of the bacterial sample administered in a single dose.

In subsequent experiments, each bacterial sample administered to mice was prepared in the same manner.

The PBS buffer solution is a product of FUJIFILM Wako Pure Chemical Corp. (https://labchem-wako.fujifilm.com/jp/product/detail/W01WO0116-2355.html) and has the following composition.

pH: 7.1-7.3
NaCl: 9000 mg/L
$KH_2PO_4$: 144 mg/L
$Na_2HPO_4$ (anhydrous): 421 mg/L

The same PBS buffer was used in subsequent experiments.

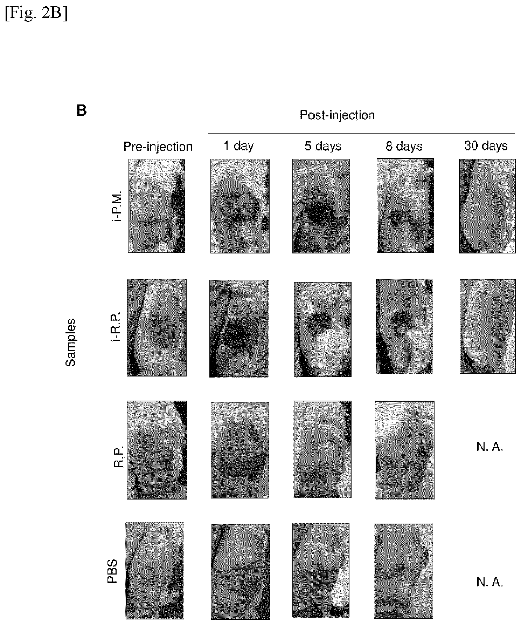

Fig. 2B shows photographs of mice after intravenous administration of each bacteria.

As shown in the figure, the tumor size of the mice administered with i-R.P. or i-P.M. decreased over time, and the tumors had disappeared 30 days after the treatment.

On the other hand, the mice administered with commercial RP or PBS buffer did not show any reduction in tumor size, but instead the tumors grew larger over time, with the tumor volume exceeding 2000 mm$^3$. Therefore, the experiment was stopped for these mice from the viewpoint of humane endpoint. Accordingly, no photographs were taken 30 days after treatment.

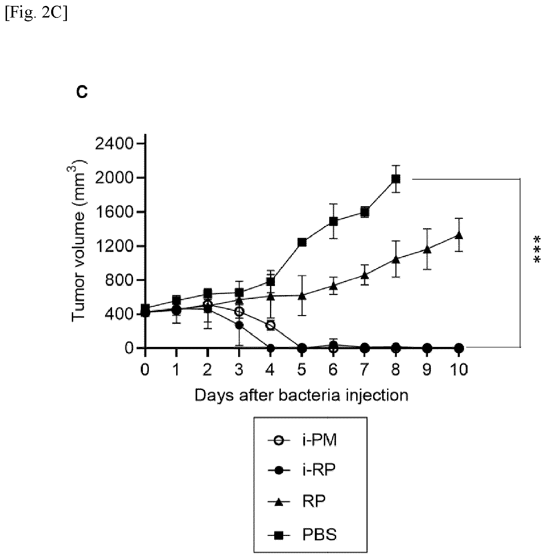

Fig. 2C shows the results of evaluating the antitumor activity of various bacteria (N=4). The figure shows the change in solid tumor volume in each group. When PBS or RP was administered, the volume of the solid tumor increased. On the other hand, when i-R.P. or i-P.M. was administered, the volume of the solid tumor decreased, and the volume became zero 4 to 5 days after administration.

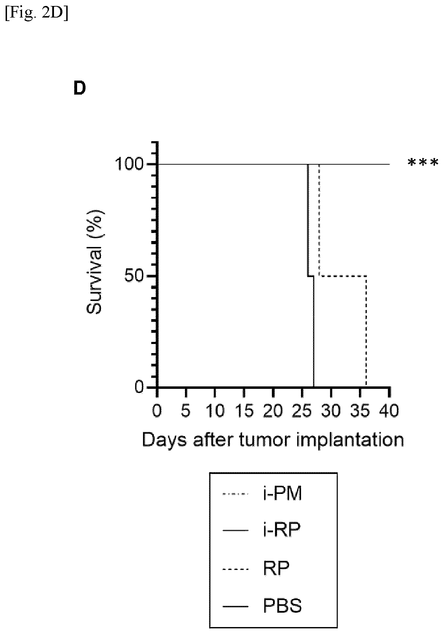

Fig. 2D shows the survival rate of mice (N=4) for 40 days after cancer cell transplantation for each bacterial administration. The survival rate of mice administered with i-P.M. or i-R.P. was 100%. On the other hand, the survival rate of mice administered with PBS or RP was 0% within 40 days.

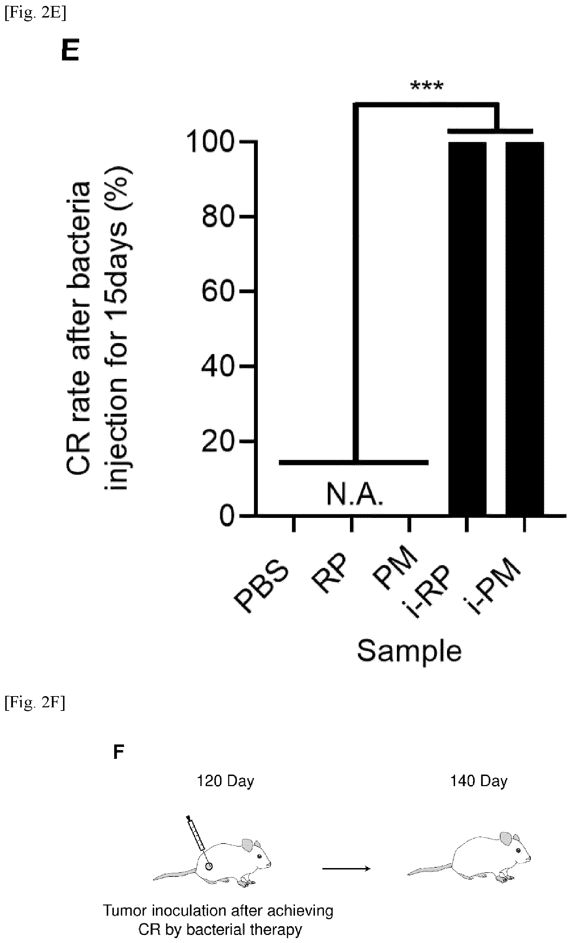

Fig. 2E shows the complete response (CR) achievement rate for each experimental group. The groups administered with i-P.M. or i-R.P. recorded a high CR achievement rate. However, the mice administered with the commercially available PM showed extreme weakness and gait disturbance, so the experiment was immediately stopped from the viewpoint of humane endpoint. Therefore, no data was available for the commercially available PM.

Fig. 2F shows the experimental method for investigating the acquisition of cancer immunity. As shown in the figure, mice that achieved CR by administration of bacteria (i-P.M. or i-R.P.) were re-implanted with Colon-26 120 days after the achievement of CR. The progress was observed for 20 days after the re-implantation.

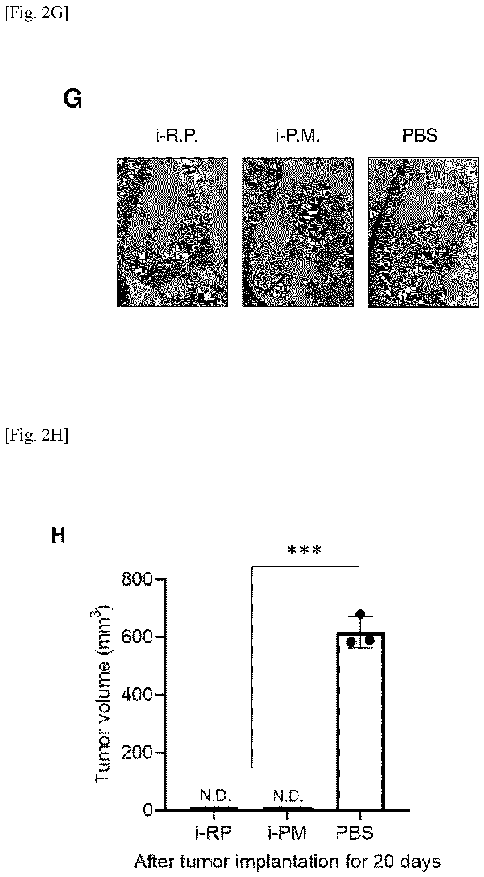

Fig. 2G shows a photograph showing the results of the observation. In the photograph, the black arrow indicates the site of cancer cell transplantation, and the black dashed line indicates the site where the tumor had formed. In mice that achieved CR using i-P.M. and i-R.P., the re-transplanted Colon-26 did not take root and no tumors were formed. On the other hand, tumor formation was observed when Colon-26 was transplanted into healthy mice administered PBS. In addition, Fig. 2H shows the measurement results of the tumor volume (N=3) for each treatment group. In this figure, N.D. means that no tumor formation was observed.

From these results, it is believed that bacteriotherapy using i-P.M. or i-R.P. is very useful for tumor treatment. That is, it is believed that the bacteria isolated from the tumor have excellent antitumor activity and are very useful for tumor treatment. For example, the bacteria isolated from the tumor may have antitumor activity that shrinks or eliminates the tumor formed in the colon cancer model mouse within 30 days after administration of the bacteria to the mouse, and in particular, may have antitumor activity that shrinks or eliminates the tumor within 10 days after administration.

These results also suggest that the toxicity of i-P.M. and i-R.P. is very low. For example, the bacteria isolated from the tumor have low toxicity, and the survival rate of the mice for 40 days after administration of the bacteria to the colon cancer model mice may be, for example, 90% or more, 95% or more, or 100%.

These results also suggest that bacteriotherapy with i-P.M. or i-R.P. can be used to acquire cancer immunity. In other words, administration of bacteria isolated from tumors not only treats tumors but also acquires immunity against tumors.

<Histological staining>

The antitumor mechanism of various bacteria was analyzed by histoimmunostaining of tumor sections after bacterial administration. Specifically, each 200 $\mu$L of a bacterial sample of i-P.M. ($5 \times 10^8$ CFU/mL), a bacterial sample of i-R.P.($5 \times 10^9$ CFU/mL), or a PBS buffer was injected into the tail vein of a colon cancer model mouse (female, 8 weeks; n=5; average weight= 19 g; average tumor size up to 400 mm$^3$; BALB/cCrSlc; Japan SLC) inoculated with Colon26. Twenty four hours after administration, the tumors were excised and paraffin sections of the tumors were prepared. The paraffin sections were histologically stained and observed under an optical microscope. The histological staining was performed by Biopathology Research Institute Co., Ltd.

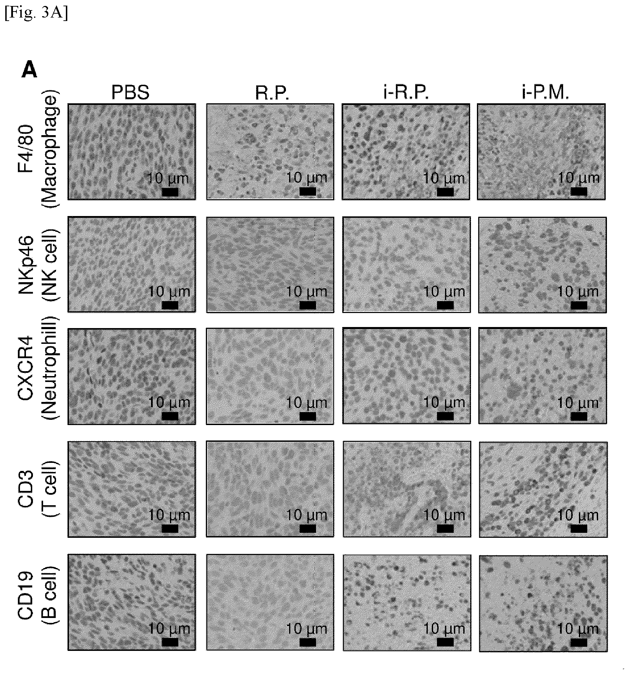

The results of immune cell staining are shown in Fig. 3A. As shown in the figure, it is observed that i-P.M. significantly activates both innate immunity (macrophages, NK cells, neutrophils) and adaptive immunity (T cells, B cells). In addition, although i-R.P. does not have as high an immune stimulation ability as i-P.M., it does activate macrophages, T cells, and B cells significantly. On the other hand, no such activation was observed with PBS and RP.

In other words, bacteria isolated from tumors have the ability to activate immune cells, and this activity is thought to be one of the factors that results in excellent antitumor activity.

In addition, the bacteria isolated from a tumor may be used to activate immune cells, particularly in tissues in which a tumor is present. That is, the present invention also provides an immune cell activating agent comprising the bacteria isolated from a tumor.

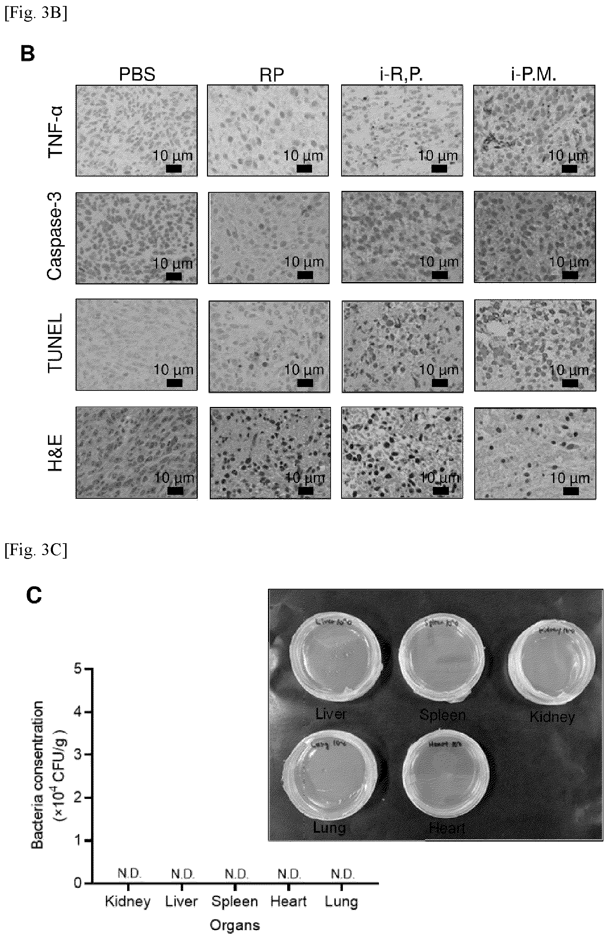

The staining results of antitumor biomarkers are shown in Fig. 3B. As shown in the figure, both i-P.M. and i-R.P. induced the expression of the necrosis marker TNF-$\alpha$ and the apoptosis marker Caspase-3, and significant tissue damage was observed. i-P.M. induced particularly strong expression of TNF-$\alpha$ and Caspase-3.

In other words, bacteria isolated from tumors have the effect of enhancing the expression of antitumor biomarkers, and this effect is thought to be one of the factors that result in excellent antitumor activity.

Furthermore, the bacteria isolated from a tumor may be used to enhance the expression of an antitumor biomarker, particularly in tissues in which a tumor is present. That is, the present invention also provides an antitumor biomarker expression enhancer comprising the bacteria isolated from a tumor.

<In vivo toxicity>

The in vivo toxicity of bacteria (i-R.P., i-P.M.) was investigated by performing mouse blood tests and tissue analysis of various organs.

For mouse blood testing, a bacterial sample of various bacteria (i-R.P., i-P.M.)($5 \times 10^6$ CFU/mL) or a PBS buffer was administered in 200 $\mu$L each to the tail vein of 10-week-old female mice (n=5; average weight = 21 g, BALB/cCrSlc, Japan SLC). Thirty days after administration, blood was collected from the abdominal aorta. Complete blood cell count (CBC) and biochemical tests were analyzed by Japan SLC, Inc. and Oriental Yeast Co., Ltd.

For tissue identification of various organs, a bacterial sample of various bacteria (i-R.P., i-P.M.)(i-R.P. = $5 \times 10^9$ CFU/mL, i-P.M. =$5 \times 10^8$ CFU/mL) or a PBS buffer was administered in 200 $\mu$L each into the tail vein of colon cancer model mice bearing solid tumors (up to 400 mm$^3$). Thirty days after administration (when complete response (CR) was achieved), various organs were excised from the mice. Paraffin sections were prepared from the excised organs. Each paraffin section was histologically stained with hematoxylin and eosin (H&E) and observed under an optical microscope. Histological staining was performed by Biopathology Research Institute Co., Ltd.

Tables 1 and 2 below show the results of blood tests when i-R.P. and i-P.M. were administered.

[Table 1]

Table 1: Complete blood count (CBC) and biochemical parameters 30 days after administration of PBS (200 μL) or i-R.P. (200 μL, 5×10⁶ CFU/mL) to mouse tail vein

| Measured value | Entry | Unit | PBS (n = 5) | i-RP (n = 5) | P value |
|---|---|---|---|---|---|
| CBC | WBC | $\times 10^2$ /μL | 56.00 ± 7.07 | 56.33 ± 7.57 | > 0.05 |
| | RBC | $\times 10^4$ /μL | 865.80 ± 19.25 | 876.20 ± 50.79 | > 0.05 |
| | HGB | g/dL | 12.58 ± 0.37 | 12.66 ± 0.81 | > 0.05 |
| | HCT | % | 40.74 ± 0.79 | 40.82 ± 2.40 | > 0.05 |
| | MCV | fL | 47.08 ± 0.71 | 46.58 ± 0.53 | > 0.05 |
| | MCH | pg | 14.52 ± 0.26 | 14.44 ± 0.15 | > 0.05 |
| | MCHC | g/dL | 30.09 ± 0.49 | 31.00 ± 0.33 | > 0.05 |
| | PLT | $\times 10^4$/μL | 67.46 ± 4.97 | 68.10 ± 5.05 | > 0.05 |
| Biochemical parameters | TP | g/dL | 4.10 ± 0.13 | 4.00 ± 0.18 | > 0.05 |
| | ALB | g/dL | 2.90 ± 0.09 | 2.70 ± 0.13 | > 0.05 |
| | BUN | mg/dL | 21.02 ± 3.61 | 21.94 ± 2.77 | > 0.05 |
| | CRE | mg/dL | 0.10 ± 0.01 | 0.10 ± 0.01 | > 0.05 |
| | Na | mEq/L | 151.40 ± 1.34 | 152.40 ± 1.52 | > 0.05 |
| | K | mEq/L | 3.38 ± 0.28 | 3.14 ± 0.36 | > 0.05 |
| | Cl | mEq/L | 118.20 ± 1.10 | 118.80 ± 0.84 | > 0.05 |
| | AST | IU/L | 64.00 ± 8.49 | 67.60 ± 11.37 | > 0.05 |
| | ALT | IU/L | 34.50 ± 4.95 | 35.40 ± 3.98 | > 0.05 |
| | LDH | IU/L | 284.25 ± 60.32 | 264.80 ± 56.14 | > 0.05 |
| | AMY | IU/L | 1806.00 ± 157.14 | 1853.20 ± 193.67 | > 0.05 |
| | CK | IU/L | 211.50 ± 50.35 | 205.67 ± 71.46 | > 0.05 |

The ± in the results indicates the standard deviation of five experiments. Statistical analysis was performed based on Student's t-test.

Abbreviation: ALB, albumin; ALT, alanine transaminase; AMY, amylase; AST, aspartate aminotransferase; BUN, blood urea nitrogen; Cl, chlorine; CK, creatine kinase; CRE, creatinine; CRP, C-reactive protein; HCT, hematocrit; HGB, hemoglobin; K, potassium; LDH, lactate dehydrogenase; MCH, mean corpuscular hemoglobin; MCHC, mean corpuscular hemoglobin concentration; MCV, mean corpuscular volume; Na, sodium; PLT, platelet; RBC, red blood cell; TP, total protein; WBC, white blood cell.

[Table 2]

Table 2: CBC and biochemical parameters 30 days after administration of PBS (200μL) or i-P.M. (200μL, 5×10⁶ CFU/mL) to mouse tail vein

| Measured value | Entry | Unit | PBS (n = 5) | i-PM (n = 5) | P value |
|---|---|---|---|---|---|
| CBC | WBC | $\times 10^2$/μL | 66.2 ± 12.70 | 66.8 ± 12.13 | > 0.05 |
| | RBC | $\times 10^4$/μL | 948.8 ± 8.70 | 883.8 ± 31.58 | > 0.05 |
| | HGB | g/dL | 14.1 ± 0.31 | 13.88 ± 0.46 | > 0.05 |
| | HCT | % | 44.7 ± 0.91 | 40.98 ± 1.67 | > 0.05 |
| | MCV | fL | 47.1 ± 0.81 | 46.38 ± 0.27 | > 0.05 |
| | MCH | pg | 14.8 ± 0.19 | 15.7 ± 0.12 | > 0.05 |
| | MCHC | g/dL | 31.5 ± 0.40 | 33.84 ± 0.24 | > 0.05 |
| | PLT | $\times 10^4$/μL | 72.6 ± 4.67 | 70.76 ± 6.38 | > 0.05 |
| | TP | g/dL | 4.1 ± 0.15 | 3.9 ± 0.16 | > 0.05 |
| | ALB | g/dL | 2.7 ± 0.15 | 2.6 ± 0.06 | > 0.05 |
| | BUN | mg/dL | 21.5 ± 2.04 | 22.0 ± 1.85 | > 0.05 |

(continued)

Table 2: CBC and biochemical parameters 30 days after administration of PBS (200μL) or i-P.M. (200μL, $5\times10^6$ CFU/mL) to mouse tail vein

| Measured value | Entry | Unit | PBS (n = 5) | i-PM (n = 5) | P value |
|---|---|---|---|---|---|
| | CRE | mg/dL | $0.13 \pm 0.01$ | $0.12 \pm 0.03$ | > 0.05 |
| | Na | mEq/L | $153.0 \pm 1.14$ | $151.0 \pm 0.55$ | > 0.05 |
| | K | mEq/L | $3.6 \pm 0.42$ | $3.6 \pm 0.14$ | > 0.05 |
| Biochemical parameters | Cl | mEq/L | $117.2 \pm 0.45$ | $117.8 \pm 1.30$ | > 0.05 |
| | AST | IU/L | $51.4 \pm 6.43$ | $58.8 \pm 10.43$ | > 0.05 |
| | ALT | IU/L | $33.2 \pm 7.36$ | $34.6 \pm 6.58$ | > 0.05 |
| | LDH | IU/L | $244.2 \pm 75.88$ | $254.6 \pm 68.68$ | > 0.05 |
| | AMY | IU/L | $1671.4 \pm 97.66$ | $1682.8 \pm 131.58$ | > 0.05 |
| | CK | IU/L | $181.6 \pm 113.95$ | $182.6 \pm 128.41$ | > 0.05 |

The $\pm$ in the results indicates the standard deviation of five experiments. Statistical analysis was performed based on Student's t-test.

Abbreviation:ALB, albumin; ALT, alanine transaminase; AMY, amylase; AST, aspartate aminotransferase; BUN, blood urea nitrogen; Cl, chlorine; CK, creatine kinase; CRE, creatinine; CRP, C-reactive protein; HCT, hematocrit; HGB, hemoglobin; K, potassium; LDH, lactate dehydrogenase; MCH, mean corpuscular hemoglobin; MCHC, mean corpuscular hemoglobin concentration; MCV, mean corpuscular volume; Na, sodium; PLT, platelet; RBC, red blood cell; TP, total protein; WBC, white blood cell.

As shown in these tables, there was no significant difference in the blood test results between the cases where i-R.P. was administrated or the cases where i-P.M. was administered and the cases where a PBS buffer was administered. In other words, it is considered that neither administration of i-R.P. nor administration of i-P.M. has an adverse effect on the state of the blood.

H&E histological staining of the various organs when i-P.M., i-R.P., or PBS were administered are shown in Fig. 3E. As shown in the figure, no damage to organ tissues due to bacterial administration was observed.

These results suggest that administration of bacteria isolated from tumors is not toxic in terms of blood conditions and the conditions of various organs.

<Colony assay>

The remaining status of bacteria in various tissues after bacterial administration was investigated by the following colony assay. That is, each 200 μL of a bacterial sample of i-P.M. ($5\times10^8$ CFU/mL), a bacterial sample of i-R.P. ($5\times10^9$ CFU/mL) or a PBS buffer was injected into the tail vein of a colon cancer model mouse (female, 8 weeks; n=3; average weight = 19 g; average tumor size up to 400 mm$^3$; BALB/cCrSlc; Japan SLC) inoculated with Colon26. Thirty days after administration (attainment of CR), each tissue was excised, cut, and its weight was measured. Each tissue was homogenized in a PBS buffer at 4°C using a pestle. The mixture obtained by the homogenization was shaken at 15°C and 380 rpm/min for 20 minutes. After the shaking, the supernatant of the mixture was diluted 10-fold with a PBS buffer to obtain a sample. Each sample (100 μL) was inoculated on an agar medium and anaerobically cultured for 7 days to form bacterial colonies on the agar medium. The number of bacterial colonies was counted visually (manually).

The results of the colony assay when i-R.P. was administered are shown in Fig. 3C. The figure confirmed that no i-R.P. remained in the organs after cancer treatment.

The results of the colony assay when i-P.M. was administered are shown in Fig. 3D. It was confirmed from the figure that no i-P.M. remained in the organs after cancer treatment.

These results indicate that the bacteria isolated from the tumor do not persist in various tissues when administered.

<Effects on other cancers>

As shown in Fig. 4A, sarcoma model mice were generated in the same manner as the colon cancer model mice. To generate sarcoma model mice, mouse sarcoma (sarcoma)-derived cells (Meth-A) were subcutaneously transplanted. Six to 10 days after the transplantation, 200 μL of a bacterial sample of i-P.M. ($1\times10^8$ CFU/mL) was injected into the tail vein of mice (n=3) that had formed solid tumors (up to 100 mm$^3$), and tumors were observed over a period of 14 days.

The time course of the tumor is shown in Fig. 4B. As shown in the figure, CR was achieved after intravenous administration of i-P.M. and i-R.P. Furthermore, no cancer recurrence was observed after the 8th day.

These results indicate that bacteria isolated from the tumor exhibit antitumor activity not only against colon cancer but also against sarcomas such as sarcoma.

As shown in Fig. 4C, metastatic lung cancer model mice were generated. That is, 4-week-old female wild-type mice (n=3; average weight= 15 g; C57BL/6NCrSlc) were purchased from Japan SLC, Inc. and acclimated for one week. Mouse melanoma cell (B16-F10 cell) dispersion liquid ($5\times10^6$ cells) (200 μL) was administered to the tail vein of the mice. In this way, metastatic lung cancer model mice were generated. One week after the administration, bacterial samples (i-R.P., i-P.M.) of various bacteria (i-R.P.=$5\times10^9$ CFU/mL, i-P.M. =$5\times10^8$ CFU/mL) or a PBS buffer was administered in 200 μL each to the tail vein of metastatic lung cancer model mice. Seven days after administration, the lungs were excised, photographed, and weighed. In addition, untreated mice (healthy mice) that were not administered mouse melanoma cells or various bacteria were also prepared.

Fig. 4D shows a photograph of the excised lungs. As shown in the figure, the lungs of the untreated mice and the mice administered with i-R.P. or i-P.M. were of similar size. On the other hand, tumors were confirmed in the mice administered with PBS. In other words, it can be seen that the administration of various bacteria caused the tumors to go into remission.

The weights of the lungs excised from the model mice after each treatment and from the untreated mice (N=3) are shown in Fig. 4E. The lungs of the i-R.P. or i-P.M.-administered groups had the same weight as the healthy lungs of the untreated mice, whereas the PBS-administered group showed an increase in lung weight associated with tumor formation.

Fig. 4F shows the changes in body weight of the model mice and untreated mice after each treatment (N=3). The arrows in the figure indicate the timing of administration of PBS or bacteria. Weight gain can be observed over time in the untreated mice, but a significant weight loss is observed from around day 10 in the PBS-administered group. A slight weight loss is observed in all bacteria-administered groups the day after administration, but healthy weight gain is observed thereafter.

These results indicate that bacteria isolated from tumors exhibit antitumor activity not only against colon cancer but also against lung cancer.

<Analysis of optical property of bacteria>

The absorption spectrum of a bacterium dispersion liquid in which i-R.P. isolated from a tumor or commercial R.P. was dispersed was measured at room temperature using a UV-Vis-NIR spectrophotometer (V-730 BIO; Jasco). The bacterium dispersion liquid was a dispersion liquid in which each bacterium was dispersed in a PBS buffer at a concentration of $2\times10^7$ CFU/mL.

The dispersion liquid was prepared as follows.

i-R.P. was anaerobically cultured in Cys-free 543 ATCC medium at 26°C-30°C for 3 days while irradiating with a tungsten lamp. The culture solution obtained by the anaerobic culture was centrifuged at 3000 rpm for 5 minutes. The bacteria precipitated by the centrifugation were suspended in a PBS buffer to obtain a bacterial suspension. The number of bacteria in the suspension was measured using a microorganism counter (Panasonic, model number NP-BCM01-A), and based on the measurement results, an appropriate amount of a PBS buffer was added to the suspension to obtain a predetermined bacterial concentration, thereby obtaining a dispersion liquid of i-R.P. at the above concentration.

RP was anaerobically cultured for 3 days at 26°C-30°C in 543 ATCC medium supplemented with Cys while irradiating with a tungsten lamp. The culture solution obtained by the anaerobic culture was centrifuged at 3000 rpm for 5 minutes in the same manner as for i-R.P., and the bacteria precipitated by the centrifugation were suspended in a PBS buffer, and a PBS buffer was then added appropriately to obtain a predetermined bacterial concentration, and a dispersion liquid of R.P. with the above concentration was obtained.

The measurement conditions for the UV-Vis-NIR spectrophotometer measurement were as shown in Table 3 below.

[Table 3]

Table 3: Measurement conditions

| Measurement mode | Bandwidth | Scan speed | Response | Data interval |
|---|---|---|---|---|
| Abs | 1 nm | 1000 nm/min | Quick | 0.5 nm |

| Start wavelength | End wavelength | Vertical scale | Scanning mode | Accumulation/ Repetition |
|---|---|---|---|---|
| 1100 nm | 300 nm | 1-0 | Continuous | Accumulation, 3 times |

In addition, the fluorescence spectrum of a bacterium dispersion liquid in which i-R.P. isolated from a tumor or commercial R.P. was dispersed was measured using fluorescence spectrometers (FP-8600 NIR Spectrofluorometer; Jasco). The excitation wavelength was 805 nm. The bacterial dispersion liquid was a dispersion liquid in which the bacterium was dispersed in a PBS buffer at a concentration of $2.5 \times 10^7$ CFU/mL. The dispersion liquid was prepared as described above. The measurement conditions for the fluorescence spectrometer measurement were as shown in Table 4 below.

[Table 4]

Table 4: Measurement conditions

| Measurement mode | Ex bandwidth | Em bandwidth | Response | Sensitivity |
|---|---|---|---|---|
| Emission | 10 nm | 10 nm | 1 sec | Medium |

| Vertical scale | Data interval | Scan speed | Accumulation/cycle |
|---|---|---|---|
| Auto | 1 nm | 200 nm/min | 3 |

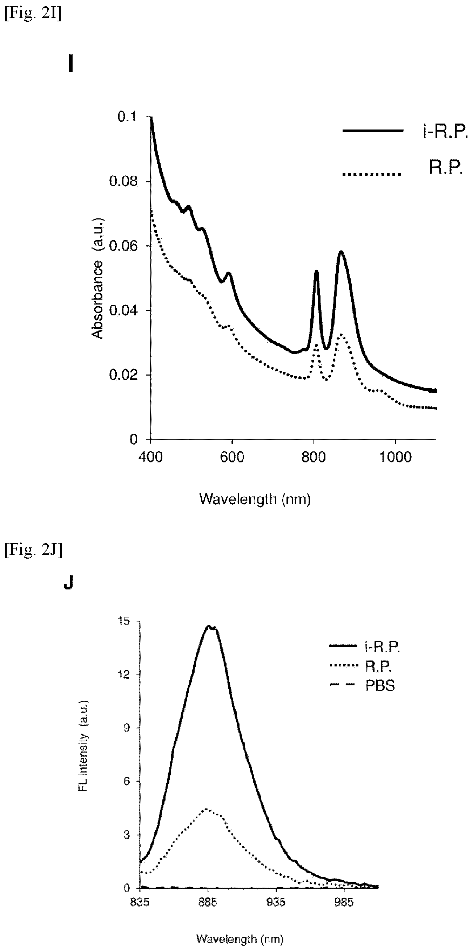

Fig. 2I shows the measurement results of the absorption spectrum. As shown in the figure, it can be seen that i-R.P. has a higher light absorption property than RP. For i-R.P., the absorbance at 808 nm is, for example, 0.029 or more, and the absorbance at 865 nm is, for example, 0.032 or more. i-R.P. has particularly high absorbance at these wavelengths. Such differences in absorbance are thought to be due to differences in the expression status of light-absorbing substances (for example, light-absorbing proteins, etc.). In addition, taking into account the experimental results on antitumor activity described above, it is considered that such high absorbance can be used as an indicator that the bacteria have antitumor activity.

The measurement results of the fluorescence spectrum are shown in Fig. 2J. As shown in the figure, it can be seen that i-R.P. has a higher fluorescence property than RP. For i-R.P., for example, the fluorescence intensity at 888 nm is 4.4 or more. i-R.P. has a particularly high fluorescence intensity at this wavelength. Such a difference in fluorescence intensity is considered to be due to a difference in the expression state of the fluorescent substance (e.g., fluorescent protein, etc.). In addition, in light of the experimental results on antitumor activity described above, such a high fluorescence intensity is considered to be usable as an indicator that the bacterium has antitumor activity.

These results indicate that bacteria isolated from tumors (particularly purple non-sulfur bacteria) have an optical property that is different from bacteria not present in tumors (e.g., commercially available bacteria). Furthermore, the optical property can be used as an indicator of antitumor activity. It is also believed that the optical property can be used to determine the presence or absence of tumors or to identify the location or shape of tumors.

The absorption spectrum and the fluorescence spectrum of a bacterium dispersion liquid in which tumor-isolated i-R.P. or commercial R.P. was dispersed were measured as described above. The bacterial concentrations of the bacterial dispersion liquids used to measure the absorption spectra were $2 \times 10^7$ CFU/mL for both cases. The bacterial concentrations of the bacterial dispersion liquids used to measure the fluorescence spectra were $2.5 \times 10^7$ CFU/mL for both cases. and the excitation wavelength was 805 nm.

The dispersion liquids were prepared as follows.

i-P.M. was anaerobically cultured for 3 days at 26°C-30°C in 543 ATCC medium supplemented with Cys while irradiating with a tungsten lamp. The culture solution obtained by the anaerobic culture was centrifuged at 3000 rpm for 5 minutes. The bacteria precipitated by the centrifugation were suspended in a PBS buffer to obtain a bacterial suspension. The number of bacteria in the suspension was measured using a microorganism counter (Panasonic, model number NP-BCM01-A), and based on the measurement results, an appropriate amount of a PBS buffer was added to the suspension to obtain a predetermined bacterial concentration, thereby obtaining a dispersion liquid of i-P.M. at the above concentration.

PM was anaerobically cultured in 802 NBRC medium at 30°C for 3 days. The culture solution obtained by the anaerobic culture was centrifuged at 3000 rpm for 5 minutes in the same manner as for i-P.M., and the bacteria precipitated by the centrifugation were suspended in a PBS buffer, and a PBS buffer was then added appropriately to obtain a predetermined bacterial concentration, and a dispersion liquid of P.M. with the above concentration was obtained.

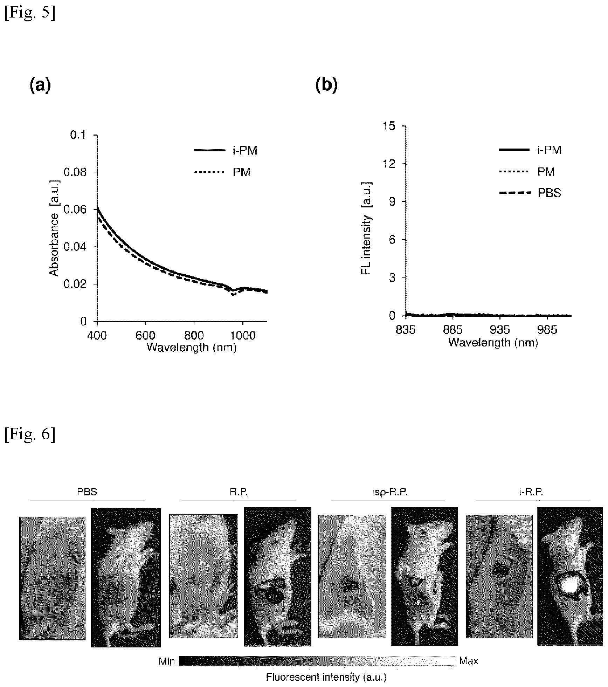

The measurement results are shown in Fig. 5, where (a) is the measurement result of the absorption spectrum and (b) is the measurement result of the fluorescence spectrum. These results show that the light absorption properties of i-P.M. and commercial PM are the same, and that the fluorescence properties of i-P.M. and commercial PM are also the same.

<In vivo fluorescence bioimaging>

To measure the biodistribution of bacteria using near-infrared (NIR) fluorescence, a bacterial sample of R.P. (200 $\mu$l of $5 \times 10^9$ CFU/mL liquid, i.e. $1 \times 10^9$ CFU) or a bacterial sample of i-R.P. (200 $\mu$l of $5 \times 10^9$ CFU/mL liquid, i.e. $1 \times 10^9$ CFU) or a PBS buffer without bacteria was injected once into the tail vein of a colon cancer model mouse (female, 8 weeks; n=3; average weight = 19 g; average tumor size up to 400 mm$^3$; BALB/cCrSIc; Japan SLC) inoculated with Colon26. Near-infrared fluorescence images of the whole mouse body were observed using an imaging system (VISQUE (trademark) In Vivo Smart-LF, Vieworks). The excitation and fluorescence wavelengths for the observation were $\lambda_{ex}$ =740-790 nm and $\lambda_{em}$ =810-860 nm, respectively. CleVue (trademark) software (Vieworks) was used for image analysis.

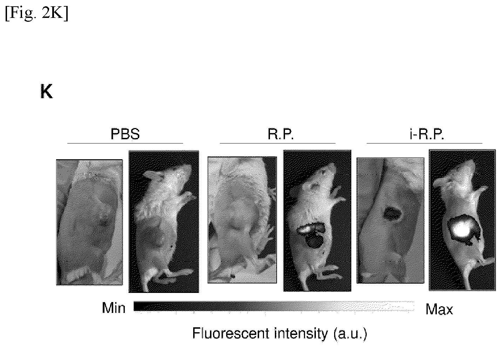

The results of the observation are shown in Fig. 2K. The image shown in the figure was taken 4 days after the administration. From the image shown in the figure, it can be seen that i-R.P. exhibits higher tumor specificity and a higher fluorescent property compared to R.P.

<Statistical analysis of data>

In the data, $\pm$ indicates standard deviation, and n indicates the number of samples used. Statistical analysis of the data was performed using Student's t-test. *, **, and *** indicate p values of < 0.05, < 0.005, and < 0.001, respectively.

<Complex bacterium and purely-isolated bacterium>

As mentioned above, "i-R.P." is a complex bacterium consisting of two species, Rhodopseudomonas Palustris and Proteus mirabilis, isolated from tumors. "isp-R.P." is only Rhodopseudomonas Palustris isolated from tumors, i.e., Rhodopseudomonas Palustris purely-isolated from tumors. Their antitumor activities and optical property were evaluated.

For this evaluation, in addition to the isp-RP and i-R.P. bacterial samples, a PBS buffer and commercial RP bacterial samples were also prepared. The i-R.P. and RP bacterial samples were prepared as described above. The isp-RP bacterial sample was prepared by the same method as the i-R.P. bacterial sample.

As described in the above <In vivo anti-cancer experiment>, colon cancer model mice were created, and the four samples were administered once into the tail vein of the mice with solid tumors (up to 400 mm$^3$) in the same manner. The condition after administration was observed, and fluorescence was observed using the imaging system as described in the above <In vivo fluorescence bioimaging>.

The results of these observations are shown in Fig. 6. The figure shows photographs and images obtained by fluorescent observation for each sample.

As shown in the photographs in the figure, both i-R.P. and isp-RP have antitumor activity. On the other hand, the antitumor activity of commercially available RP, as seen in i-R.P. and isp-RP, was not confirmed. Also, no antitumor activity was confirmed in PBS.

As shown in the fluorescent observation images in the figure, it was confirmed that both i-R.P. and isp-R.P. were accumulated in the tumor. It was also confirmed that both i-R.P. and isp-RP had the fluorescent property described above. On the other hand, fluorescence was confirmed for the commercially available RP, but it was weaker than the fluorescence from i-R.P. and isp-RP. No fluorescence was confirmed for PBS.

The antitumor activity of i-R.P. was stronger than that of isp-RP. Also, the fluorescence intensity of i-R.P. was stronger than that of isp-RP. Therefore, it is considered that the composite bacteria consisting of purple non-sulfur bacteria and other bacteria are superior to the purely-isolated purple non-sulfur bacteria in terms of antitumor activity and optical property.

Furthermore, as a result of further investigation into the ratio of the complex bacteria, it was found that it is preferable for Rhodopseudomonas Palustris to be contained in a greater amount than Proteus mirabilis, and for example, it was found that a composition ratio based on CFU of Rhodopseudomonas Palustris and Proteus mirabilis of 99:1 to 55:45, particularly 99:1 to 60:40, is preferable for exerting excellent antitumor activity.

<Base sequence analysis>

16S ribosomal RNA gene of isp-RP was sequenced. The sequence was as shown in the following sequence ID No. 1.

SEQ ID No. 1:

```
AGCGAACGCTGGCGGCAGGCTTAACACATGCAAGTCGAACGGGCGTAGCAATACGTCAGTGGCAGACGGG
TGAGTAACGCGTGGGAACGTACCTTTTGGTTCGGAACAACACAGGGAAACTTGTGCTAATACCGGATAAG
CCCTTACGGGGAAAGATTTATCGCCGAAAGATCGGCCCGCGTCTGATTAGCTAGTTGGTGAGGTAATGGC
TCACCAAGGCGACGATCAGTAGCTGGTCTGAGAGGATGATCAGCCACATTGGGACTGAGACACGGCCCAA
ACTCCTACGGGAGGCAGCAGTGGGGAATATTGGACAATGGGGGAAACCCTGATCCAGCCATGCCGCGTGA
GTGATGAAGGCCCTAGGGTTGTAAAGCTCTTTTGTGCGGGAAGATAATGACGGTACCGCAAGAATAAGCC
CCGGCTAACTTCGTGCCAGCAGCCGCGGTAATACGAAGGGGGCTAGCGTTGCTCGGAATCACTGGGCGTA
AAGGGTGCGTAGGCGGGTTTCTAAGTCAGAGGTGAAAGCCTGGAGCTCAACTCCAGAACTGCCTTTGATA
CTGGAAGTCTTGAGTATGGCAGAGGTGAGTGGAACTGCGAGTGTAGAGGTGAAATTCGTAGATATTCGCA
AGAACACCAGTGGCGAAGGCGGCTCACTGGGCCATTACTGACGCTGAGGCACGAAAGCGTGGGGAGCAAA
CAGGATTAGATACCCTGGTAGTCCACGCCGTAAACGATGAATGCCAGCCGTTAGTGGGTTTACTCACTAG
TGGCGCAGCTAACGCTTTAAGCATTCCGCCTGGGGAGTACGGTCGCAAGATTAAAACTCAAAGGAATTGA
CGGGGGCCCGCACAAGCGGTGGAGCATGTGGTTTAATTCGACGCAACGCGCAGAACCTTACCAGCCCTTG
ACATGTCCAGGACCGGTCGCAGAGACGCGACCTTCTCTTCGGAGCCTGGAGCACAGGTGCTGCATGGCTG
TCGTCAGCTCGTGTCGTGAGATGTTGGGTTAAGTCCCGCAACGAGCGCAACCCCCGTCCTTAGTTGCTAC
CATTTAGTTGAGCACTCTAAGGAGACTGCCGGTGATAAGCCGCGAGGAAGGTGGGGATGACGTCAAGTCC
TCATGGCCCTTACGGGCTGGGCTACACACGTGCTACAATGGCGGTGACAATGGGAAGCTAAGGGGCGACC
CTTCGCAAATCTCAAAAAGCCGTCTCAGTTCGGATTGGGCTCTGCAACTCGAGCCCATGAAGTTGGAATC
GCTAGTAATCGTGGATCAGCATGCCACGGTGAATACGTTCCCGGGCCTTGTACACACCGCCCGTCACACC
ATGGGAGTTGGCTTTACCTGAAGACGGTGCGCTAACCAGCAATGGGGGCAGCCGGCCACGGTAGGGTCAG
CGACTGGGGTG
```

The base sequence is 100% homologous to and has 100% sequence identity with the 16S ribosomal RNA gene of the commercially available RP. Thus, at least in terms of the gene, isp-RP is identical to the commercially available RP.

Genetic mutations are not expected to occur during the isolation from tumors and culture in culture media described above. Therefore, the isp-RPs are expected to be genetically identical to commercially available RPs.

On the other hand, as mentioned above, the optical properties of isp-R.P. and i-R.P. are different from those of commercially available RPs, i.e., the absorption and fluorescence spectra are different. The optical properties of isp-R.P. and i-R.P. that are different from those of commercially available RPs are considered to be acquired by being isolated from tumors and then cultured in a medium. For example, the optical properties are considered to be acquired by increasing the expression of light-absorbing substances and fluorescent substances (particularly light-absorbing proteins and fluorescent proteins) by the process of isolation from the tumor (particularly the process of isolation and culture in a medium).

In addition, it is believed that isp-RP and i-R.P. have acquired excellent antitumor activity as a result of acquiring the optical property. Therefore, it is believed that the possession of the optical property can be used as an indicator of the possession of excellent antitumor activity. In addition, the isolation process from the tumor (particularly the isolation process and the culture process) performed to acquire the optical property is believed to be a useful process for imparting antitumor activity to bacteria.

The 16S rRNA gene sequence of i-P.M. was analyzed. The base sequence was as shown in the following sequence ID No. 2.

SEQ ID No. 2:

```
ATTGAACGCTGGCGGCAGGCCTAACACATGCAAGTCGAGCGGTAACAGGrAGAAAGCTTGCTTTCTTGCT
GACGAGCGGCGGACGGGTGAGTAATGTATGGGGATCTGCCCGATAGAGGGGGGATAACTACTGGAAACGGT
GGCTAATACCGCATAATGTCTACGGACCAAAGCAGGGGGCTCTTCGGACCTTGCACTATCGGATGAACCCA
TATGGGATTAGCTAGTAGGTGGGGTAAAGGCTCACCTAGGCGACGATCTCTAGCTGGTCTGAGAGGATGA
TCAGCCACACTGGGACTGAGACACGGCCCAGACTCCTACGGGAGGCAGCAGTGGGGAATATTGCACAATG
GGCGCAAGCCTGATGCAGCCATGCCGCGTGTATGAAGAAGGCCTTAGGGTTGTAAAGTACTTTCAGCGGG
GAGGAAGGTGATAAGGTTAATACCCTTrTCAATTGACGTTACCCGCAGAAGAAGCACCGGCTAACTCCGT
GCCAGCAGCCGCGGTAATACGGAGGGTGCAAGCGTTAATCGGAATTACTGGGCGTAAAGCGCACGCAGGC
GGTCAATTAAGTCAGATGTGAAAGCCCCGAGCTTAACTTGGGAATTGCATCTGAAACTGGTTGGCTAGAG
TCTTGTAGAGGGGGGTAGAATTCCATGTGTAGCGGTGAAATGCGTAGAGATGTGGAGGAATACCGGTGGC
GAAGGCGGCCCCCTGGACAAAGACTGACGCTCAGGTGCGAAAGCGTGGGGAGCAAACAGGATTAGATACC
CTGGTAGTCCACGCTGTAAACGATGTCGATTTAGAGGTTGTGGTCTTGAACCGTGGCTTCTGGAGCTAAC
GCGTTAAATCGACCGCCTGGGGAGTACGGCCGCAAGGTTAAAACTCAAATGAATTGACGGGGGCCCGCAC
AAGCGGTGGAGCATGTGGTTTAATTCGATGCAACGCGAAGAACCTTACCTACTCTTGACATCCAGCGAAT
CCTTTAGAGATAGAGGAGTGCCTTCGGGAACGCTGAGACAGGTGCTGCATGGCTGTCGTCAGCTCGTGTT
GTGAAATGTTGGGTTAAGTCCCGCAACGAGCGCAACCCTTATCCTTTGTTGCCAGCACGTrATGGTGGGA
ACTCAAAGGAGACTGCCGGTGATAAACCGGAGGAAGGTGGGGATGACGTCAAGTCATCATGGCCCTTACG
AGTAGGGCTACACACGTGCTACAATGGCAGATACAAAGAGAAGCGACCTCGCGAGAGCAAGCGGAACTCA
TAAAGTCTGTCGTAGTCCGGATTGGAGTCTGCAACTCGACTCCATGAAGTCGGAATCGCTAGTAATCGTA
GATCAGAATGCTACGGTGAATACGTTCCCGGGCCTTGTACACACCGCCCGTCACACCATGGGAGTGGGTT
GCAAAGAAGTAGGTAGCTTAACCTTCGGGAGGGCGCTTACCACTTTGTGATTCATGACTGGGGTG
```

The base sequence is 99.80% homologous to and 99% sequence identity with the 16S rRNA gene sequence of commercially available PM. Thus, the genes of i-P.M. are considered to be substantially identical to those of commercially available PM from a genetic standpoint.

Genetic mutations are not expected to occur during the isolation from tumors and culture in culture medium described above. Therefore, i-P.M. is also believed to be genetically identical to commercially available P.M.

On the other hand, as mentioned above, i-P.M. has superior antitumor activity compared to commercially available PM. This is believed to be acquired by the isolation process from the tumor (particularly the isolation process and the culture process in a medium). In addition, the isolation process from the tumor (particularly the isolation process and the culture process) performed to obtain the superior optical property of antitumor activity is believed to be a useful process for imparting antitumor activity to bacteria.

The present invention also provides the following use, product, and method. Each component of the use, product, and method, such as the "antitumor agent" and the "bacteria isolated from a tumor", is as explained above, and the explanation also applies to the use, product, and method.

[1] Use of bacteria isolated from a tumor for the production of an antitumor agent.
[2] A bacterium isolated from a tumor, used for the production of an antitumor agent.
[3] A method for treating a tumor, comprising administering a bacterium isolated from a tumor.

Accession number

[0277]

Proteus mirabilis (Super Bac): NITE BP-03626
Complex bacteria of Rhodopseudomonas Palustris and Proteus mirabilis (Musashi): NITE BP-03627

Claims

1.   An antitumor agent comprising a purple non-sulfur bacterium isolated from a tumor.

2.   The antitumor agent according to claim 1, wherein the purple non-sulfur bacterium is a bacterium of the genus Rhodopseudomonas or a bacterium of the genus Blastochloris, or both of these.

3.   The antitumor agent according to claim 1 or 2, which is administered parenterally.

4. The antitumor agent according to claim 1 or 2, further comprising, in addition to the purple non-sulfur bacteria, other bacteria isolated from tumors.

5. The antitumor agent according to claim 4, wherein the other bacteria are bacteria of the genus Proteus.

6. The antitumor agent according to claim 4, wherein the antitumor agent contains the purple non-sulfur bacteria and the other bacteria in a ratio of 1:99 to 99: 1.

7. The antitumor agent according to claim 1 or 2, wherein the bacterium contained in the antitumor agent has an immune cell stimulation property.

8. The antitumor agent according to claim 1 or 2, wherein the bacterium contained in the antitumor agent has an expression enhancing property that enhances the expression of an antitumor biomarker in a tumor.

9. The antitumor agent according to claim 1 or 2, wherein the bacterium contained in the antitumor agent exhibits the following property regarding the absorption spectrum:
<Property regarding absorption spectrum> when measuring the absorption spectrum of a dispersion liquid in which the bacterium contained in the antitumor agent is dispersed in a PBS buffer at a concentration of $2\times10^7$ CFU/mL, the absorbance at 808 nm is 0.029 or more.

10. The antitumor agent according to claim 1 or 2, wherein the bacterium contained in the antitumor agent exhibits the following property regarding the fluorescence spectrum:
<Property regarding fluorescence spectrum> when measuring the fluorescence spectrum of a dispersion liquid in which the bacterium contained in the antitumor agent is dispersed in a PBS buffer at a concentration of $2.5\times10^7$ CFU/mL, the fluorescence intensity at 888 nm is 4.4 or more.

11. A method for generating an image for tumor identification, comprising:

    an administration step of administering one or more types of bacteria isolated from a tumor to an animal; and
    an image generation step of capturing an image of the animal after the administration while irradiated with light in a predetermined wavelength range to generate an image for identifying any tumors that may be present in the animal.

12. A bacterium isolated from a tumor, comprising at least a purple non-sulfur bacterium.

13. The bacterium according to claim 12, wherein the bacterium isolated from a tumor further comprises a bacterium of the genus Proteus.

14. The bacterium according to claim 12 or 13, wherein the purple non-sulfur bacterium is a bacterium of the genus Rhodopseudomonas or a bacterium of the genus Blastochloris, or both.

15. The bacterium according to claim 12 or 13, which exhibits the following property regarding the absorption spectrum:
<Property regarding absorption spectrum> when measuring the absorption spectrum of a dispersion liquid in which the bacterium is dispersed in a PBS buffer at a concentration of $2\times10^7$ CFU/mL, the absorbance at 808 nm is 0.029 or more.

16. The bacterium according to claim 12 or 13, which exhibits the following property regarding the fluorescence spectrum:
<Property regarding fluorescence spectrum>
when measuring the fluorescence spectrum of a dispersion liquid in which the bacterium is dispersed in a PBS buffer at a concentration of $2.5\times10^7$ CFU/mL, the fluorescence intensity at 888 nm is 4.4 or more.

17. A bacterium production method, comprising:

    an isolation step of isolating bacteria from a tumor; and
    a culturing step of culturing the bacteria isolated in the isolation step in a medium not containing cysteine added;
    wherein the cultured bacteria comprise at least a purple non-sulfur bacterium and have antitumor activity.

[Fig. 1]

[Fig. 2A]

A

[Fig. 2B]

[Fig. 2C]

C

[Fig. 2D]

**D**

[Fig. 2E]

**E**

[Fig. 2F]

**F**

120 Day                    140 Day

Tumor inoculation after achieving
CR by bacterial therapy

[Fig. 2G]

## G

i-R.P.      i-P.M.      PBS

[Fig. 2H]

## H

[Fig. 2I]

**I**

[Fig. 2J]

**J**

[Fig. 2K]

**K**

[Fig. 3A]

**A**

[Fig. 3B]

[Fig. 3C]

[Fig. 3D]

[Fig. 3E]

[Fig. 4A]

[Fig. 4B]

## B

Post-injection

[Fig. 4C]

[Fig. 4D]

**D** No-treatments (Healthy lung)    i-R.P.    i-P.M.    PBS
1 cm    1 cm    1 cm    1 cm

[Fig. 4E]

[Fig. 4F]

F

[Fig. 5]

(a)

(b)

[Fig. 6]

Fluorescent intensity (a.u.)

## INTERNATIONAL SEARCH REPORT

| International application No. |
|---|
| **PCT/JP2023/017320** |

### A. CLASSIFICATION OF SUBJECT MATTER

*A61K 35/74*(2015.01)i; *A61K 50/00*(2006.01)i; *A61P 35/00*(2006.01)i; *A61P 37/04*(2006.01)i; *C12N 1/20*(2006.01)i
FI:    A61K35/74 A; A61K50/00 200; A61P35/00; A61P37/04; C12N1/20 A ZNA

According to International Patent Classification (IPC) or to both national classification and IPC

### B. FIELDS SEARCHED

Minimum documentation searched (classification system followed by classification symbols)

A61K35/74; A61K50/00; A61P35/00; A61P37/04; C12N1/20

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | YANG, X. et al. Optically activatable photosynthetic bacteria-based highly tumor specific immunotheranostics. Nano Today. 15 February 2021, vol. 37, no. 101100, pp. 1-14 abstract, page 5, left column, first paragraph to page 6, left column, first paragraph, page 6, right column, first paragraph to page 9, right column, first paragraph, page 10, right column, sixth paragraph, page 12, left column, third paragraph, fig. 3A-C, fig. 4B-D, fig. 5 | 1-3, 7, 8, 11-16 |
| Y | | 4-6 |
| A | | 9, 10, 17 |
| X | WO 2022/025043 A1 (JAPAN ADVANCED INSTITUTE OF SCIENCE AND TECHNOLOGY) 03 February 2022 (2022-02-03) fig. 18 | 11, 12 ,14 |
| A | | 1-10, 13, 15-17 |
| Y | MURATA T. et al. Oncolytic effect of Proteus mirabilis upon tumor bearing animal. Life Sci. April 1965, vol. 4, no. 10, pp. 1055-1067 fig. 1, 2, tables 5-9 | 4-6 |
| A | | 1-3, 7-17 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **12 July 2023** | **01 August 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT

| | International application No. |
|---|---|
| | **PCT/JP2023/017320** |

### C. DOCUMENTS CONSIDERED TO BE RELEVANT

| Category* | Citation of document, with indication, where appropriate, of the relevant passages | Relevant to claim No. |
|---|---|---|
| A | XIE, G.J. et al. Photo-fermentative bacteria aggregation triggered by L-cystein during hydrogen production. Biotechnol. Biofuels. 03 May 2013, vol. 6, no. 64, pp. 1-14 <br>     abstract | 1-17 |
| P, X | GOTO, Y. et al. Discovery of intratumoral oncolytic bacteria toward targeted anticancer theranostics. Adv. Sci. 07 May 2023, no. 2301679, pp. 1-14 <br>     whole document | 1-17 |

Form PCT/ISA/210 (second sheet) (January 2015)

**INTERNATIONAL SEARCH REPORT**

International application No.

**PCT/JP2023/017320**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    "In the form of an Annex C/ST.25 text file" above should be understood as "in ST.26 format".

Form PCT/ISA/210 (continuation of first sheet) (January 2015)

## INTERNATIONAL SEARCH REPORT

International application No.

**PCT/JP2023/017320**

| Box No. III | Observations where unity of invention is lacking (Continuation of item 3 of first sheet) |
|---|---|

This International Searching Authority found multiple inventions in this international application, as follows:

(Invention 1) Claims 1-10
    Document 1 (Nano Today, 15 February 2021, vol. 37 no. 101100, pp. 1-14) describes an antitumor agent containing Rhodopseudomonas palustris. Claims 1-3 are indistinguishable from the invention described in document 1 and lack novelty, and thus do not have a special technical feature. However, claim 4, which depends from claim 1, has the special technical feature of "further comprising other bacteria isolated from a tumor in addition to a purple non-sulfur bacterium". Claims 5-6 also have the same technical feature as claim 4. Thus, claims 1-6 are classified as invention 1.
    Claims 7-10 depend from claim 1, which is classified as invention 1, and are therefore classified as invention 1.

(Invention 2) Claim 11
    Claim 11 is an independent claim, and document 1 discloses a purple non-sulfur bacterium that has been isolated from a tumor, and therefore cannot be said to share the same or corresponding technical features with claim 1, which is classified as invention 1.
    Therefore, claim 11 cannot be classified as invention 1, and is thus is classified as invention 2.

(Invention 3) Claims 12-16
    Claim 12 is an independent claim, and in light of the disclosures of document 1, cannot be said to share the same or corresponding technical features with claim 1, which is classified as invention 1, or claim 11, which is classified as invention 2.
    Therefore, claim 12 cannot be classified as invention 1 or invention 2, and is therefore classified as invention 3.
    Claims 13-16 are dependent on claim 12 and are therefore classified as invention 3.

(Invention 4) Claim 17
    Claim 17 is an independent claim, and in light of the disclosures of document 1, cannot be said to share the same or corresponding technical features with claim 1, which is classified as invention 1, claim 11, which is classified as invention 2, or claim 12, which is classified as invention 3.
    Therefore, claim 17 cannot be classified as invention 1-3, and is therefore classified as invention 4.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
    ☐ The additional search fees were accompanied by the applicant's protest and, where applicable, the payment of a protest fee.
    ☐ The additional search fees were accompanied by the applicant's protest but the applicable protest fee was not paid within the time limit specified in the invitation.
    ☐ No protest accompanied the payment of additional search fees.

Form PCT/ISA/210 (continuation of first sheet) (January 2015)

**INTERNATIONAL SEARCH REPORT**
Information on patent family members

International application No.

**PCT/JP2023/017320**

| Patent document cited in search report | Publication date (day/month/year) | Patent family member(s) | Publication date (day/month/year) |
|---|---|---|---|
| WO 2022/025043 A1 | 03 February 2022 | (Family: none) | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022025043 A
- WO 01162355 A

**Non-patent literature cited in the description**

- **MASAYUKI YOKOYAMA**. *Drug Delivery System*, 2018, vol. 33 (2), 89-97
- **HISATAKA KOBAYASHI**. *Drug Delivery System*, 2014, vol. 29 (4), 274-284
- **SHIBIN ZHOU et al.** *Nature Reviews Cancer*, 2018, vol. 18, 727-743